Europäisches Patentamt

European Patent Office

Office européen des brevets

(11) Publication number: **0 107 070**
**B1**

# EUROPEAN PATENT SPECIFICATION

(45) Date of publication of patent specification: 17.11.88

(51) Int. Cl.⁴: **C 12 Q 1/04**

(21) Application number: 83109554.2

(22) Date of filing: 26.09.83

(54) Detection of microbial pathogens.

(30) Priority: 30.09.82 US 431776
23.08.83 US 525164

(43) Date of publication of application:
02.05.84 Bulletin 84/18

(45) Publication of the grant of the patent:
17.11.88 Bulletin 88/46

(84) Designated Contracting States:
BE CH DE FR GB IT LI LU NL

(56) References cited:
US-A-3 671 399
US-A-4 212 948

CHEMICAL ABSTRACTS, vol. 81, no. 5, 5th
August 1974, page 223, no. 23021u, Columbus,
Ohio, US, M. HALL et al.: "Detection of
bacteremia with liquid media containing
sodium poly(anetholsulfonate)

(73) Proprietor: Wadley Technologies, Inc.
9000 Harry Hines Boulevard
Dallas, Texas 75235 (US)

(72) Inventor: **Dorn, Gordon Lee**
**1232 Lausanne**
**Dallas Texas 75208 (US)**

(74) Representative: UEXKÜLL & STOLBERG
**Patentanwälte**
**Beselerstrasse 4**
**D-2000 Hamburg 52 (DE)**

## Description

### Technical field

This invention relates to a novel method and article for detecting microbial pathogens. In another aspect, this invention relates to a novel technique and means for selectively separating microorganisms from a sample fluid which contains antimicrobial factors. In still another aspect, this invention relates to a method and means for use in the detection of microbial pathogens which provides improved recovery of microorganisms. In yet another aspect, this invention relates to a method and means for accurately quantitating the number of microorganisms present in a sample fluid at a given time when quantitated at a later time.

### Background art

Septicemia, which is the presence of pathogenic microorganisms in the blood, is one of the most serious types of infections encountered. There is unanimous agreement in the medical profession that septicemia is second only to meningitis in terms of serious infections. Even though modern medicine has provided an armament of antibiotics and fungal drugs, the mortality rate from septicemia is approximately twenty-five percent. Also, when shock accompanies septicemia, the mortality rate increases to over sixty percent. Debilitating diseases, major surgery, administration of immunosuppressive drugs or anticancer medications causes patients to be particularly prone to septicemia. Early diagnosis of the causative agent in conjunction with the use of the appropriate antibiotic therapy is essential in fighting septicemia. Consequently, it is imperative that the physician know as rapidly as possible, not only that the patient has septicemia, but also the identity of the affecting microorganisms and the susceptibility of the microorganisms to antibiotic agents. Thus, proper and timely diagnosis of septicemia depends upon very rapid and efficient quantitative analysis of the patient's blood. Further, it is necessary during the quantitative analysis of the patient's blood that the blood sample not be contaminated with pathogens from the hospital environment.

Uremia, which is the presence of pathogenic microorganisms in the urine, occurs most commonly in infants, pregnant women, patients with obstructive lesions or following instrumentation of the urinary tract or with urologic diseases affecting micturation. Uremia can result in a localized infection within the bladder or kidneys. When confined to the bladder, the infection is usually well controlled by antimicrobial therapy. Once the kidneys are infected, however, lesions may continue to progress despite treatment leading to chronic pylonephritis and septicemia.

Various analytical systems have been utilized to determine the presence of microorganisms in a body fluid. The conventional systems include the liquid broth culture technique, the so-called pour plate method and the filtration method using a solid matrix filter. Each of these systems has its drawbacks and none of the systems provide a rapid detection of microorganisms in the blood sample. Generally, the liquid broth method is not quantitative, and the pour plate method and filtration method (using a solid matrix filter) are open systems subject to external contamination, e.g., the introduction of pathogens into the culture by the laboratory atmosphere and personnel.

In United States Patent 3,671,399 a system for use in the determination of microbes in a body fluid is described, which contains an alkali metal salt of polyanetholsulfonate together with polyanionic compounds. The system allows the microbes to replicate while natural bacteriastatic components and certain antibiotics are inhibited and coagulation is prevented.

Recently developed method and apparatus for quantitatively analyzing pathogens from a sample fluid are disclosed in United States Patent No. 4,131,512 entitled "Method of Detecting Microbial Pathogens Employing a Cushioning Agent" and its division U.S. Patent No. 4,212,948 entitled "Apparatus For Detecting Microbial Pathogens Employing A Cushioning Agent". The technique disclosed in the above patents involves (when analyzing a blood sample) pre-lysis of corpuscular compounds followed by centrifugation to concentrate the microorganisms away from the other constituents including antimicrobial factors present in the blood. The concentrated microorganisms are then placed upon a nutrient media such that the sample is diluted a minimum of sixtyfold. It has been previously documented that this technique yielded more positive cultures than the conventional liquid broth culture, the pour plate method, or the filtration method using the solid matrix filter. Gordon Dorn, Jeoffrey A. Land, and George E. Wilson, 1979 Improved Blood Culture Technique Based on Centrifugation: Clinical Evaluation. *J. Clinical Microbiology* 9:391—396. While the lysis-centrifugation technique is undoubtedly an improvement in analysis of microbial pathogens, the potential disadvantage of this technique is that the specimen is exposed to antimicrobial factors present in the blood sample such as serum factors and antibiotics prior to centrifugation and subsequent distribution on appropriate growth media such as agar plates. In general, if any antimicrobial factors present in the sample are inhibiting and not cidal to the microorganisms, then any such factors which are present with the microbial pathogens during the centrifugation step would generally become sufficiently diluted when deposited on the growth media to prevent inhibiting of the growth of the microorganisms. By practicing the above described lysis-centrifugation technique, however, there are certain fastidious microorganisms whose growth can be affected by certain antibiotics when diluted in accordance with the above lysis-centrifugation technique. Of more importance, if the antimicrobial factors, such as antibiotics, are cidal, then their presence within the tube prior to centrifugation and placement on

EP 0 107 070 B1

the growth media can result in destruction of the microorganisms during this time and valuable positive cultures could be missed. Alternately undesirable growth of the microorganisms could occur during that time which would effect the accuracy of quantitation. It has been recently recognized that removal of antimicrobial factors can be accomplished without substantially altering the microflora in the specimen by using a mixed resin bed system to remove various antimicrobial factors. Such resin bed systems are disclosed in U.S. Patent No. 4,145,304 and U.S. Patent No. 4,174,277. However, the use of such resin beds subjects the sample to additional manipulations and possible contamination.

Of the systems available for prevention of growth activities of antimicrobials in urine, significant time lag problems exist.

While these conventional systems include means for inhibiting growth of the microorganisms, their use inherently includes undesirable time lags between the time the sample is collected and deposited within the system which can result in undesirable growth of microorganisms before inhibition. In addition, no antibiotic inhibiting or blocking means is provided in these systems. Thus, the practice of the improvement of the subject invention wherein the antimicrobial factor deactivation system is present in the cup at the time of micturation preserves the microbial status of the urine sample by instantly blocking the known delterious action of bactericidal antibiotics. The practice of the improvement of the subject invention furthermore acts as a bacteriostatic agent thus preserving the actual count of microorganisms in the presence or absence of bactericidal agents.

Therefore, a closed sterile method and means is needed for receiving a fluid sample suspected of containing microbial pathogens and antimicrobial factors which will provide for a liquid state deactivation of the antimicrobial factors during the time that the sample is isolated so that once the sample is removed from the vessel and placed on growth media, the microorganisms present in the sample including the fastidious microorganisms will proliferate and become identifiable.

Disclosure of the invention

According to the invention, there is provided an improved microbial pathogen detection technique which includes a system for deactivating antimicrobial factors and maintaining the microbial quantitative integrity within a sample fluid after it has been collected and before the microbial pathogens are analyzed.

According to a preferred embodiment of the subject invention, a sample fluid soluble system for deactivating microbial factors within a sample fluid containing said antimicrobial factors and microbial pathogens and method of use thereof, is provided which serves the following purposes:

(1) immediate blockage of the cidal action of penicillins, cephalosporins, and aminoglycosides,

(2) initiation of an anaerobic transport system for fastidious organisms susceptible to the lethal action of oxygen,

(3) complete neutralization of the cidal action of normal human blood, and

(4) hold stable the viable count of microorganisms over a period of time.

The procedure can be utilized on all types of body fluids such as blood, bone marrow, spinal and pleural fluids, body secretions, urine and the like. Generally, when employed in connection with a blood sample, the system for deactivating antimicrobial factors will include a lysing agent. The novel liquid system of the subject invention can be utilized in a sample collection or transporting container and allowed to be admixed with the sample after it has been collected but before microbial pathogens therein are analyzed such as, for example, by depositing them upon a growth media for microbial pathogens. The novel system of the subject invention can be in the form of an aqueous solution contained within said sample collections and transporting container. However, the novel system for deactivating antimicrobial factors is preferably positioned in said container in the form of solid particles which are soluble in the sample fluid.

It is envisioned that the subject invention can be utilized within the lysis-centrifugation devices such as disclosed in U.S. Patent 4,212,948 issued July 15, 1980 and entitled "Apparatus For Detecting Microbial Pathogens Employing A Cushioning Agent", which employs the basic method disclosed in U.S. Patent 4,131,512 issued December 26, 1978 entitled "Method For Detecting Microbial Pathogens Employing A Cushioning Agent". Also, in accrdance with one embodiment of the subject invention, a novel method of assembling and sterilizing a lysis-centrifugation device is provided which includes:

(a) depositing a liquid cushioning agent such as disclosed in said '948 patent, and an antimicrobial deactivation system in the form of solid particles within a lysis-centrifugation tube;

(b) creating a vacuum in said tube and heating said tube to the vaporization temperature of said liquid cushioning agent, e.g., about 120°C for a sufficient time, e.g., about 30 minutes to sterilize the interior of said tube and thereafter cooling said tube to room temperature.

In addition, the system of the subject invention can be utilized in practicing the lysis-centrifugation technique as disclosed in U.S. Patent 4,164,449 issued August 14, 1979 and entitled "Surface Separation Technique For The Detection Of Microbial Pathogens".

Surprisingly the novel system of the subject invention will inhibit growth of microbial pathogens which are contained within the sample fluid during the period of time that they are contained within the container such as the lysis-centrifugation tube while the tube is being held for processing. Such system will allow hold times of up to 24 hours without undesirable growth occurring within the tube which could result in excessive count and hence false clinical interpretation once the sample is deposited on growth media.

3

The antimicrobial factor deactivating system of the subject invention contains extremely high concentrations of specific chemical compounds which serve to neutralize antibiotics and/or normal human serum factors. This elevated concentration cannot readily be incorporated in conventional broth systems because the high concentrations of chemicals required would prove inhibitory to many potentially pathogenic organisms. Thus, the antimicrobial factor deactivating system of the subject invention will effectively deactivate antibiotics and other antimicrobial factors where a sample fluid is mixed therewith, and it is necessary that the resulting mixture be diluted on growth media for the microbial pathogens, for example, to concentrations of the deactivating chemicals which are noninhibitory to the pathogenic organisms. Thus, the invention is particularly useful and advantageously employed in any system in which dilution is necessary prior to processing such as sputums, urines, and the lysis-centrifugation system disclosed in U.S. Patents 4,164,449; 4,131,512; and 4,221,948 described above because of the high dilution factor inherent in the lysis-centrifugation system.

Therefore, the practice of the lysis-centrifugation system as described above but including the antimicrobial factor deactivation system within the centrifugation tube for treating the blood sample prior to centrifugation and deposit of the concentrated microorganisms on the media will protect the microorganisms from attack by antimicrobial factors which are present in the liquid sample such as antibiotics and antiserum factors which are cidal in nature. If such microbial pathogens are destroyed within the centrifugation tube or transport system, then undesirable false negative cultures or inaccurate quantitation may result. The basic benefit of use of the subject invention can be more graphically illustrated by the following theory. Septicemia, microorganisms in the bloodstream with clinical signs of shock, DIC, hypotension, etc., does not imply that the bloodstream itself is infected. In this theoretical model, there is primary infection elsewhere such as the kidneys, a lung, or the like, and the microorganisms are being seeded at a given rate into the bloodstream. The immune system and/or antibiotics are eliminating the microorganisms at a fixed rate. A patient survives a septic crisis if and only if the seeding rate is less than the rate of clearance. Thus, based upon this theoretical model, conventional blood culture systems will yield a significant number of false negative cultures because once the specimen is drawn, microbial seeding from the primary source ceases, but the antimicrobial factors present in the patient's blood are still active. Hence, during transport, or processing, these factors may kill the viable organisms that were present at the time of draw, and therefore, render the test negative. This concept becomes especially important for immunologically competent patients and those who are on a broad spectrum bactericidal antibiotics. Thus, the practice of the improvement of the subject invention in conjunction with the lysis-centrifugation system is to literally preserve the microbial status of the blood sample by instantly blocking the known deleterious action of the immune system and bactericidal antibiotics prior to dilution of these factors on agar plates which is an inherent feature in the lysis-centrifugation method.

The employment of the antimicrobial factor deactivation system in urine analysis will involve the presence of the antimicrobial factor deactivation system in the micturation receptacle from which a clinically appropriate aliquot of urine may be removed for direct microbial analysis. Thus, the practice of the subject invention is to literally preserve the microbial status of the urine sample by instantly blocking the known deleterious action of bactericidal antibiotics and by acting as a bacteriostatic agent even in the absence of antimicrobial agents.

Brief description of drawings

This invention can be more easily understood from the study of the drawings in which:

Figure 1 is cross-sectional view of a centrifugation article which can be used to practice the subject invention;

Figures 2—9 depict steps of a method for detecting microbial pathogens which can employ the subject invention; and

Figure 10 depicts another embodiment of the subject invention which comprises a device for collecting and transporting body secretion samples.

Detailed description

The novel antimicrobial factor deactivation system of the subject invention includes specific chemical agents at relatively high concentrations which will deactivate antimicrobial factors such as antibiotics and the cidal agents within normal human blood, in accordance with the invention. Specified blocking agents for aminoglycoside antibiotics and polymixin B are included within the antimicrobial factor deactivation system. Typical aminoglycoside antibiotics include gentamicin, tobramycin and amikacin. The aminoglycosides and polymixin B all have net positive charges. When this charge is blocked, these compounds lose their potency. Therefore, in accordance with this invention, a blocker for this positive charge is included within the antimicrobial factor deactivation system. A preferred compound is sodium polyanetholsulfonate. The sodium polyanetholsulfonate will inhibit the action of aminoglycosides and polymixin B in direct proportion to its concentration and these antibiotics are completely inhibited at a concentration of approximately 0.6% by weight of sodium polyanetholsulfonate. Another such blocker compound deactivation system of the subject invention contains sufficient sodium polyanetholsulfonate or sodium amylosulfate to result in between about 0.3% by weight to about 1.0% by weight and preferably from about 0.3% by weight to about 0.6% by weight based upon the total weight of sample fluid and antimicrobial factor deactivation system if sodium polyanetholsulfonate or sodium amylosulfate are used alone. Sodium polyanetholsulfonate and sodium amylosulfate are known to be inhibitory to certain microorganisms and this inhibitory effect is eliminated by subsequent dilution on growth media to an approximate final concentration of 0.01% by weight sodium polyanetholsulfonate or sodium amylosulfate on the medium.

The antimicrobial factor deactivation system of the subject invention preferably also contains the chemical composition or compositions which will inhibit the action of penicillin, streptomycin, and cephalosporins. Such chemicals are preferably selected from thioglycolate, glutathione, and N-acetyl-cysteine. These compounds can generally be present in the antimicrobial factor deactivation system to result in amounts from about 0.5 to about 6% by weight thereof on the combined sample fluid and antimicrobial factor deactivation system. The antimicrobial factor deactivation system of the subject invention can also contain cysteine for inactivating the cidal action of penicillins, cephalosporins and some aminoglycosides. The cysteine is preferably present in the antimicrobial factor deactivation system in an amount to result in from about 0.05 weight percent to about 2.5% by weight thereof and preferably from about 0.1% by weight to about 1.6% by weight thereof in the combined sample fluid and antimicrobial factor deactivation system. In a particularly preferred embodiment, the antimicrobial factor deactivating system of the subject invention contains a synergistic mixture of thioglycolate and cysteine with cysteine contained therein in an amount from about 0.05% by weight to about 2.5% by weight thereof and thioglycolate contained therein in an amount from about 0.05% by weight thereof to about 1.6% by weight thereof. This combination will deactivate the penicillins, cephalosporins and some aminoglycosides very effectively and also reduce the viscosity of the thus formed system. Thioglycolate and similar compounds by themselves cause an undesirable increase in viscosity of the antimicrobial factor deactivation system. It has been found, however, that the above-described combination of cysteine and thioglycolate results in much lower viscosity after lysing of blood, for example. In addition, the combination allows proportions of thioglycolate that are less toxic to the fastidious microorganisms. Another advantage is that cysteine is easily oxidizable and the presence of thioglycolate helps maintain the cysteine in a reduced state during the preparation of the lysis centrifugation tube. An example of the combination of the cysteine and thioglycolate that can be used in a centrifugation tube as set forth in U.S. Patent No. 4,212,948 includes an initial concentration of cysteine of 1.2 weight percent thereof and thioglycolate at 0.1% by weight of sample fluid and antimicrobial factor deactivation system and once finally diluted on growth media as disclosed in said patent a final concentration of cysteine of 0.018% by weight thereof and thioglycolate of 0.002% by weight thereof. It is noted that because of the propensity of the cysteine to oxidize, it is desirable to add the cysteine during the manufacture of a centrifugation tube during the last step prior to tube evacuation and autoclaving. The purity of the cysteine is important. Because of the high concentration of cysteine required in the antimicrobial factor deactivation system, this compound should have a purity of greater than 95%. If one uses cysteine which is contaminated with cystine, the cystine will precipitate out during the processing of blood. Since, the cystine precipitate resembles small colonies of microorganisms on the agar plate, this is an undesirable property. The inclusion of the thioglycolate and cysteine combination has a secondary affect in that it will protect anaerobic microorganisms, e.g., clostridial species, from being poisoned by the oxygen present in the blood specimen during transport. This is due to the fact that the thioglycolate and other sulfhydryl compounds are excellent oxygen scavengers. Cysteine is much more effective than other sulfhydryl compounds in blocking the cidal action of penicillins, cephalosporins and some aminoglycosides on a gram or molar basis, and as mentioned, an additional benefit of the presence of the cysteine is that it will reduce the viscosity of the lysed blood which improves the sedimentation of the microorganisms in a centrifugation tube.

In accordance with another embodiment of the subject invention, deactivators for sulfa compounds are present in the antimicrobial factor deactivation system. The sulfa compounds exert their antimicrobial action by interfering with the folic acid pathway of bacteria. This pathway is essential for the synthesis of the nucleic acids which are the primary compounds of microbial DNA. Accordingly, preferably para-aminobenzoic acid (PABA) is added to the antimicrobial factor deactivation system as a competitive inhibitor of sulfa compounds. The preferred concentration of PABA is in the range of from 5 micrograms per milliliter to about 500 micrograms per milliliter and the most preferred range is in the range of from about 10 micrograms per milliliter to about 100 micrograms per milliliter of the combined sample and antimicrobial factor deactivation system. It is noted, in general, that if the hold period for a sample fluid is 2 hours or less, then in general, the PABA compound is not necessary. However, it is known that the cidal action of sulfa drugs requires multiplication of the sensitive organisms; hence, the addition of PABA may prove desirable if the blood is held longer than 2 hours.

The antimicrobial factor deactivation system of the subject invention can also contain enzymes which react with and deactivate certain antibiotics, for example, beta-lactamase, and penicillinase. Usually from about 1 to about 20 units of activity of such enzymes will be effective in the system.

In the combinations described above the amount of polyanetholsulfonate or sodium amylosulfate is from about 0.1% by weight to about 1.0% by weight, preferably from about 0.3 to 0.6% by weight based upon the total weight of sample fluid and deactivation system.

The antimicrobial factor deactivation system of the subject invention can include other compounds, depending upon the usage of the system, for example, the system can contain lysing agents such as purified saponin disclosed in U.S. Patent 3,883,425 issued May 13, 1975 and entitled "Detoxification Of Saponins" when it is desired to process blood. The composition can also contain anticoagulant such as citrate or ethylenediamine tetraacetic acid (EDTA).

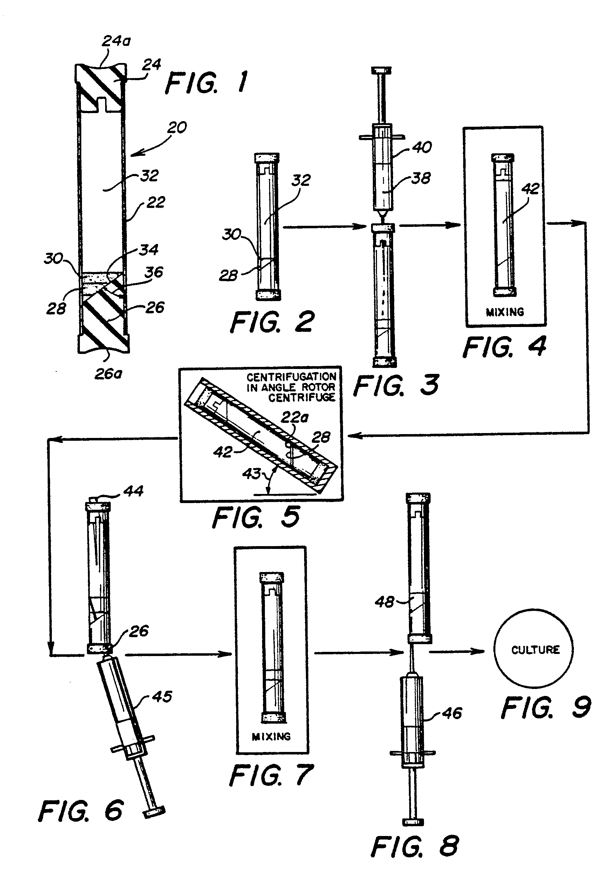

Now referring to Figure 1, centrifugation article 20 is depicted which is disclosed in the above-described U.S. Patent 4,131,512 and its division U.S. Patent 4,212,948. The incorporated patents are directed to a method and apparatus which provides for improved rapid quantitative analysis of a blood sample for the presence of microbial pathogens. The blood sample is lysed and deposited on a high density water immiscible, hydrophobic, nontoxic, liquid cushioning agent and subjected to centrifugation. The microbial pathogens contained in the lysed blood sample will collect in a layer adjacent the interface of the cushioning agent and the blood sample residue, and, in this concentrated form, can easily be separated from the residual portion of the blood sample for culturing and quantitative counting. As shown, the article 20 comprises an elongated tubular centrifugation vessel 22 having a conventional injectable closure member 24 which sealably closes the upper end thereof, and an injectable closure member 26 which sealably closes the lower end thereof. Article 20 contains an effective amount of cushioning agent 28. The antimicrobial pathogen deactivating system when utilized in elongated tubular centrifugation vessel 22 is deposited as layer 30 of particulate solid on cushioning agent 28. The antimicrobial deactivation system can be contained within an aqueous solution within article 20, e.g., about one-half milliliter, but it is preferred that said system be in the form of solid particulate powder 30. Solid particulate powder 30 is not soluble within the liquid cushioning agent 28 and has a higher shelf stability than the liquid solution formed of the ingredients. In addition, the use of the particulate solid antimicrobial deactivation system allows a novel sterilization technique to be carried out within the interior of article 20 which will be herein described below. In the preferred embodiment of the subject invention, the antimicrobial factor deactivation system is present whether in aqueous solution or layer 30 sufficient so that when a sample fluid such as blood is deposited therein, the combination of antimicrobial factor deactivation system and blood will contain from about 0.1 to about 6% by weight thereof of sodium polyanetholsulfonate; from about 0.5 to about 2.5% by weight of cysteine; from about 0.1 to about 1.6% by weight thereof of thioglycolate; and from about 5 micrograms per milliliter to about 500 micrograms per milliliter of para-aminobenzoic acid. In addition, since this particular embodiment is used for processing blood samples, the resulting total volume will also include from about 0.02 to about 1% by weight of purified saponin and from about 0.01 to about 0.5% by weight of EDTA. When the antimicrobial factor deactivation system is in the form of an aqueous solution, the centrifugation vessel 22 will draw approximately 7.5 milliliters of blood. It is preferred that said antimicrobial factor deactivation liquid system be at least 3% by volume of the total liquid in centrifugation vessel 22 including the total quantity of the antimicrobial factor deactivation system, the sample fluid and the cushioning agent and preferably from about 5% to about 30% by volume thereof. When the antimicrobial factor deactivation system is in the form of particulate layer 30, the elongated tubular centrifugation vessel 22 will draw about 8 milliliters of blood. In the most preferred embodiment of the subject invention, layer 30 will contain 0.096 grams of cysteine; 0.008 grams of thioglycolate; 0.048 grams of sodium polyanetholsulfonate; 0.018 grams purified saponin; and 0.008 grams of EDTA. It is noted that the EDTA is not necessary to prevent blood clot formation so long as adequate amounts of sodium polyanetholsulfonate are present. For example, another satisfactory blood treating system (layer 30) contains 0.048 grams sodium polyanetholsulfonate, .08 grams cysteine, .009 grams thioglycolate and 0.019 grams purified saponin.

The combination of antimicrobial factor deactivation system and urine will preferably contain from about 0.6 percent to about 2.0 percent by weight thereof sodium polyanetholsulfonate; from about 0.5 percent to about 2.5 percent by weight thereof, free-based cysteine; about 0.1 percent by weight thereof, thioglycolate; about 2.0 percent by weight thereof, sodium bicarbonate; and from about 2.5 percent to about 4.0 percent by weight thereof, sodium chloride. The sodium bicarbonate was added to the urine antimicrobial factor deactivation system in order to adjust for the normal acidity or basicity of urine and thus attain a neutral pH. The added salt, in the form of, for example, sodium chloride, increases the bacteriostatic effect of the system in the absence of antiobiotics in the urine. A free-based cysteine, such as ICN cysteine, was substituted for the previously employed L-cysteine as the former does not produce a gaseous reaction when combined with the sodium bicarbonate buffer as seen previously in the L-cysteine-sodium bicarbonate mixture.

Centrifugation vessel 22 can be made of siliconized glass or hard plastic such as polycarbonate or polypropylene. Injectable closure members 24 and 26 can comprise rubber sealing stoppers. Injectable closure members 24 and 26 both carry indentations 24a and 26a, respectively, to enhance the ease of injection by common types of injection needles. Evacuated space 32 is maintained at a lower than atmospheric pressure at a predetermined value so that the centrifugation vessel can receive a known amount of liquid by injection through injectable enclosure member 24 without excessive pressure being built up within the interior thereof which would cause injectable closure members 24 and 26 to become dislodged from the openings within the centrifugation vessel 22.

Referring especially to injectable closure member 26 at the lower end of centrifugation vessel 22, it is noted that inner surface 34 of injectable closure member 26 is positioned at an angle with respect to the walls of centrifugation vessel 22.

It is noted that article 20 is especially designed to be utilized within an angle rotor centrifuge and that the angled inner surface 34 is a complement of the angle of the rotor. It should be noted, however, that the device of the subject invention can be utilized in a conventional swinging bucket-type centrifuge. In the latter instance, surface 34 should be perpendicular to the bottom of article 20 and is otherwise utilized in the same general manner as will be described herein below for the article 20 illustrated in Figure 1. Surface 34 should be smooth and substantially free of interstitial spaces and crevices in which microbial pathogens could be entrapped. Further, the circular sealing area around surface 34 where the material of injectable closure member 26 meets the walls of the centrifugation vessel 22 should be tightly sealed so that the interface does not provide a large circular crevice in which microbial pathogens could become lodged.

The angle of incline of smooth surface 34 with respect to the walls of centrifugation vessel 22 is determined according to the centrifugation apparatus in which article 20 is to be centrifuged.

As discussed above, when a swinging bucket-type centrifuge is utilized, surface 34 will be positioned perpendicular to the bottom of the article 20. However, when an angle rotor centrifuge is utilized, surface 34 will carry the complement of the angle of the rotor. Therefore, in general, when the rotor angle ranges from about 60° to 10°, the angle of surface 34, or angle of incline 36 within the centrifugation vessel will range correspondingly from 30° to 80°. Thus, the angle of incline, depicted by arc 36, will generally be the complement of the angle at which device 20 rests within the centrifuge during centrifugation. For example, the angle of incline 36 depicted in Figure 1 is approximately 34°. Thus, for example, when article 20 is placed in an angle rotor centrifuge in which centrifugation occurs at approximately 56°, fluids contained within article 20 will be forced against surface 34 at a substantial perpendicular angle.

The amount of cushioning agent 28 employed should be sufficient to completely cover surface 34 upon centrifugation. The amount of cushioning agent utilized can vary with the parameter of the particular system chosen, for example, the stopper design, volume of residual blood and volatility of the cushioning agent utilized. A preferred amount of cushioning agent can comprise from about 3.3% to about 40% by volume based on the volume of the cushioning agent-residual blood sample mixture which is removed from article 20 and tested for the presence of microbial pathogens.

Generally, the cushioning agent of the subject invention can comprise a high density, hydrophobic, water immiscible liquid. As noted previously, the term "high density" as used herein refers to a liquid which will not be supported by the mixture of blood and blood treating fluid or any other sample fluid suspected of containing microbial pathogens in the presence of centrifugal force. In addition, the cushioning agent should be nontoxic to microbial pathogens and relatively inert with respect to butyl rubber, silicone rubber and other types of elastomers employed in the manufacture of the injectable closure members described above. The density of the cushioning agent can be in the range of from about 1.2 grams per cubic centimeter to about 2.0 grams per cubic centimeter. Generally, fluorinated hydrocarbons having the above described characteristics and having molecular weights in the range of from about 300 to about 850 are preferred. Furthermore, fluorinated hydrocarbons having the above qualities which have a vapor pressure at 25°C (77°F) and 1 atmosphere from (0.06 psi) 0.3 mmHg to about (0.58 psi) 30 mmHg and preferably a vapor pressure approximately equal to or equal to that water. Therefore, cushioning agents having the above described qualities and boiling points of about 93°C (200°F) to about 216°C (420°F) and preferably about 106°C (225°F) to about 138°C (280°F) can be utilized. The cushioning agents preferably have specific heat at least equal to or greater than 0.2 g-cal/g°C at 25°C (77°F) and 1 atmosphere, and most preferably specific heat at least equal to or greater than water. The cushioning agent should also have a vapor pressure which will not disrupt the injectable closure means from the tube during manufacturing steps such as autoclaving, for example. Fluorinated hydrocarbons sold under the trade name Fluorinert® 3M Company of Minneapolis, Minnesota, have been found to perform well as cushioning agents. Specifically, types FC-75, FC-48, and FC-43 of the Fluorinert® series have ben found to be especially useful.

Although the exact function which such cushioning agents perform is not fully known, it is believed that they improve collection of microbial pathogens which have passed from suspension in a centrifuged blood sample in at least two ways. First, the cushioning agent serves to seal interstitial spaces, cracks and crevices both on the smooth surface 34 of the centrifugation vessel 22 and the interface between the walls of the centrifugation vessel 22 and injectable closure member 26. Thus, microbial pathogens which might otherwise become entrapped in such interstitial spaces, and therefore not recovered, are recovered with the cushioning agent 28 when it is removed from article 20. Secondly, it is believed that the cushioning agent does act to cushion the impact of microbial pathogens which are forced out of suspension in a blood sample during centrifugation. This cushioning effect reduces the danger of injury to microbial pathogens which might otherwise occur upon impact. Further, while some of the microbial pathogens may actually pass into the cushioning agent, substantially none will pass completely through it and a majority will form on its surface at the interface between the cushioning agent 28 and the blood sample and collect in a layer.

After the cushioning agent 28 has been deposited within centrifugation article 20, the antimicrobial factor deactivation system 30 for the blood may also be deposited there.

Once the antimicrobial factor deactivation system 30 has been deposited in centrifugation article 20, injectable closure member 24 can be put in place and space 32 evacuated to the desired lower than atmospheric pressure, e.g. 63,5 to 76,2 cm (25 to 30 inches) of mercury. In accordance with one embodiment, the interior of centrifugation vessel 20 is next sterilized by a novel technique. It has been found that if a centrifugation vessel is heated to the vaporization point of the Fluorinert® material

EP 0 107 070 B1

therewithin, e.g., at least about 120°C and held for a sufficient time, e.g., at least about 30 minutes, the interior of the tube and the solid particulate antimicrobial factor deactivation system 30 will become sterilized by the hot Fluorinert® vapors. Once this is done, the centrifugation vessel 20 is merely cooled to room temperature and packaged for sale, for example.

Now referring to Figures 2—9, an analysis sequence is schematically depicted illustrating a preferred embodiment of the subject invention. As an example, a procedure which is carried out in accordance with one embodiment of this invention for detection of microbial pathogens within a blood sample can be carried out conveniently with the following apparatus:

The above described centrifugation article 20 containing the cushioning agent 28 and antimicrobial factor deactivating system 30. The vessel can be of 12—14 milliliters in volume.

A sterile glass syringe and one 3,8 cm (1-1/2 inch) 5,3 μm (21 gauge) disposable hypodermic needle;

One sterile glass syringe and one 2,45 cm (1 inch) 4,57 μm (18 gauge) disposable hypodermic needle;

One 1,6 cm (5/8 inch) 6,4 μm (25 gauge) hypodermic needle with cotton inserted at its hub (used as a vent);

Two blood agar plates;

Two chocolate agar plates.

It is noted that with the exception of centrifugation article 20 or some equivalent article, various types of well-known laboratory apparatus and culture media can be used to carry out the novel process of the subject invention. It is particularly noted that the culture media set forth above are exemplary only and are generally preferred to be utilized for detecting the most commonly known microbial pathogens. The blood agar plates suggested are conventionally utilized blood agar plates which are basically sheep's blood and a base nutritional agent such as brain heart infusion, which is held together with an agar solidifying agent on a petri plate. The chocolate agar plate is designed to grow certain fastitious pathogens, e.g., hemophilus.

Thus, while various apparatus can be utilized in the method of the subject invention, the above list of apparatus and materials can be conveniently utilized in the scope of this invention in a manner set forth below.

To utilize centrifugation article 20 set forth in Figure 1 in the drawing, it is initially positioned so that injectable closure member 26 with its smooth angled surface 34 is at the lower end of article 20 so that the cushioning agent 28 antimicrobial factor deactivation solids 30 rest upon smooth angled surface 34. In practice, a mixture of cushioning agent 28 and the solid particles of antimicrobial factor deactivation system 30 may occur due to handling so that two distinct layers may not always be present. This unstable mixture of cushioning agent 28 and antimicrobial factor deactivation system 30 in no way adversely affects the method set forth herein since the solids forming system 30 will rapidly dissolve in the aqueous sample (blood) and separation of the two resulting liquid phases rapidly occurs upon centrifugation.

Next, a predetermined amount of a blood sample 38 drawn from the patient, for example, 8 milliliters of blood, is injected into the evacuated space or centrifugation article 20 as depicted in Figure 3 using a common type of syringe 40. Alternately, the sample can be drawn directly into article 20 using a standard and double needle fixture supplied with conventional vacuum blood drawing devices such as sold under the mark Vacutainer® by Becton Dickinson. Then, article 20 containing the blood sample 38, the antimicrobial factor deactivating system 30, and the cushioning agent 28 is subjected to mixing to insure that the anticoagulants, red cell lysing agent, and the antimicrobial factor deactivating system 30 are completely admixed with the blood sample 38. This mixing step is depicted schematically by Figure 4. The mixing step will insure that the antimicrobial factor deactivation system 30 containing the lysing agent will be completely admixed with and solubilized by the blood sample. This solubilizing action will assure contact between antimicrobial factors and the chemical components of the antimicrobial factor deactivation system 30 and thus assure that any pathogens contained within the blood sample 38 will be protected from antimicrobial activity.

After the blood sample 38 has been treated in this manner, centrifugation article 20 is centrifuged to cause the microbial pathogens within the treated blood sample 42 to pass out of suspension and collect adjacent the interface of the high density cushioning agent 28 and the residual of the sample fluid. Some microbial pathogens will actually be deposited upon the sidewall of centrifugation vessel 22 adjacent the high end of smooth surface 34 at point 22a. This centrifugation step is represented schematically by Figure 5. The speed and time of centrifugation can vary widely depending upon the construction material of centrifugation article 20 and the type of centrifugation apparatus. The centrifugation can be conveniently accomplished by imparting from between about 1500 to 6000 gravities and preferably from about 1500 to 3000 gravities to the centrifugation article 20 containing the treated blood sample 42 and cushioning agent 28. As depicted in Figure 5, an angle rotor centrifuge is employed which places the centrifugation article 20 at an angle of 56° for example, (depicted by arc 43) during centrifugation. Thus, if smooth angled surface 34 is at a 34° angle with respect to the interior walls of centrifugation article 20, the treated blood sample 42 and cushioning agent 28 will be forced against smooth angled surface 34 at a relatively perpendicular angle during centrifugation. It is noted that when a swinging bucket type of centrifuge is employed, centrifugation article 20 will be centrifuged at substantially 0° with respect to a horizontal surface. Thus, in such a case, the angle of surface 34 will be approximately 90° and an injectable rubber closure member having a flat inner surface can be substituted for injectable closure member 26.

Once the centrifugation step has been completed, centrifugation article 20 can be removed from the

8

centrifuge and the major portion of the treated blood sample 42 from which microbial pathogens have been separated can be removed. It is noted that, as used herein, the term "residual treated blood" or "residual blood" refers to a blood sample which has been centrifuged such that the microbial pathogens present therein have collected at the bottom of the sample, hence, leaving the "residual" portion of the sample substantially free of microbial pathogens. This step is depicted in Figure 6. To aid in ease of removal, a vent needle 44 in the form of a common hypodermic needle with cotton in its hub, for example, is injected through injectable closure member 24. A second hypodermic needle with syringe 45 attached can then be injected through injectable closure member 26 to remove a major portion of the residual treated blood sample 42 from which microbial pathogens have been separated. For example, when the centrifugation vessel has a volume of from 12 to about 14 milliliters, a 3,8 cm (1-1/2 inch) 4,6 µm (18 gauge) needle can be employed to remove all but about 1.3 to 1.7 milliliters of the treated blood sample 42. As shown, it is preferred that the major portion of the residual blood sample to be withdrawn from the interior of centrifugation vessel 22 is withdrawn at a point opposite the sidewall adjacent the upper bevel end of smooth surface 34 to avoid disturbing the layer of microbial pathogens which has formed on and within the interface of the two liquids and on the sidewall of centrifugation vessel 22 adjacent the upper end of said bevelled smooth surface 34. The majority of the residual blood is removed in this step; however, a small portion of the residual blood should be left in the centrifugation vessel 22 such that of the total fluid remaining, the cushioning agent comprises from about 3.3% to about 40.0% by volume. It is preferred that no more than about 20% by volume shall be said cushioning agent because greater quantities of said cushioning agent may deleteriously effect the morphology of microbial pathogen colonies in subsequent pathogen growth steps used in the process.

Once the major portion of the treated residual blood sample has been removed, both needles may be withdrawn from injectable closure members 24 and 26, and centrifugation article 20 is then subjected to a second mixing step depicted schematically by Figure 7. However, if desired, vent needle 44 can be left in its position through injectable closure member 24 to assist in removal of the pathogen containing fluid in a later step. The second mixing step serves to resuspend microbial pathogens which have separated from the major portion of residual treated blood sample 42 and which have formed the layer described above. Resuspension of the microbial pathogens so collected in the remaining minor portion of the residual treated blood sample 42 insures greater and more uniform recovery.

Once the mixing step has resuspended, the microbial pathogens in a minor portion of the residual treated blood sample 42, the mixture of microbial pathogens in the residual treated blood sample and the high density cushioning agent can be removed from centrifugation article 20. This step is depicted in Figure 8. As noted above, if desired, the venting hypodermic needle 44 may be inserted through injectable closure member 24 to allow easier removal of the remaining constituents. The syringe 46 with attached hypodermic needle can then be injected through injectable closure member 26 to draw out the mixture 48 of cushioning agent 28, minor remaining portion of residual blood sample 42 and microbial pathogens present therein. It is noted that particularly good recovery can be obtained if the hypodermic needle used to remove these constituents is injected at the lower end of the angled smooth surface 34. It is believed that the angle of surface 34 acts, in part, as a funnel into which the remaining fluid containing the microbial pathogens flow. This mixture 48 of high density liquid cushioning agent 28, and the remaining minor portion of the residual treated blood sample 42 with the recovered microbial pathogens should be approximately 1-1/2 milliliters of fluid. This fluid is then distributed on appropriate growth media. This step is then schematically illustrated in Figure 9 in the drawing. With the apparatus set forth above, the material can be distributed as follows:

Two blood agar plates can receive 0.4 milliliters of the aqueous solution and can be incubated at 36°C in an anaerobic environment. Two chocolate agar plates can receive 0.4 milliliters of the aqueous solution and can be incubated at 36°C in a candle jar. The growth media should be checked daily for the presence of colonies. Microbiological analysis techniques can be employed. The number of microbial pathogens in one milliliter of the blood can be determined by multiplying the number of colonies by a correction factor. This correction factor takes into consideration the recovery rate for a given organism, the volumes of blood and high density cushioning agent employed and the amount of final mixture plated. In the general example set forth above, the correction factor is 0.5.

The above procedure will result in a dilution of the remaining minor portion of the residual treated blood sample 42 to at least about 1:60 on the growth media. This will assure that any residual quantity of the chemicals within the antimicrobial factor deactivation system will be diluted sufficiently so as to not inhibit the growth of microbial pathogens therewithin. The antimicrobial factor deactivation system of the subject invention will either neutralize or inhibit cidal drugs. For example, the sodium polyanetholsulfonate will generally neutralize and the cysteine will generally inhibit. Furthermore, the effect of oxygen on cysteine after removal of the sample from centrifugation vessel 22 will destroy its inhibiting effect on microorganisms. The above described dilution procedure may be necessary to dilute drugs and/or component of the antimicrobial factor deactivation system to levels which are neither cidal nor inhibitory to the growth of microorganisms. In addition, for those antibiotics which may be present in the blood sample which exert only an inhibitory and not a cidal effect on microorganisms, the 1:60 dilution will generally prove adequate to reverse their inhibitory effect on microorganisms. An example of this class of compound is gantrisin. thus, in general, the 1:60 dilution will prevent the inhibiting of growth for most

9

microorganisms/antibiotic combinations. Nevertheless, there are certain microorganisms which are uniquely sensitive to the killing or inhibitory action of certain classes of antibiotics. For example, if one is attempting the isolation of a very sensitive strain of *S. aureus* (minimum inhibitory concentration of 0.2 micrograms per milliliter) and the blood sample contained 20 micrograms per milliliter of antibiotic not blocked by sodium polyanetholsulfonate, para-aminobenzoic acid, or cysteine-thioglycolate, the organism would not grow on a conventional agar plate (20 milliliters of media) in accordance with the above-described method which normally deposits 0.4 micrograms per milliliter of blood sample. This combination yields a final dilution of approximately 1:60. Thus, this example would yield a final concentration of 0.33 micrograms per milliliter of antibiotic throughout the plate which would indeed inhibit the subsequent growth of the *S. aureus* strain. Furthermore, the dilution of the antibiotic is not instantaneous and initially the high levels of the antibiotic on the surface of the agar plate might exert a lethal effect. To circumvent this problem and yet preserve the known advantages of the lysis-centrifugation technique improved with the novel antimicrobial factor deactivation system of the subject invention, one further modification is required: namely, a big petri plate. Clinical laboratories concurrently use a 150 mm×20 mm petri plate for testing antibiotics. This plate contains between 60 ml and 80 ml of media and has 2.25 times the surface area of a conventional 100 mm×20 mm petri plate. When one streaks the 0.4 ml blood sample uniformly on the surface of this large plate, one achieves a 2.25 fold increase in the diffusion rate and a final dilution between about 1:200 to about 1:270. In the example used above, this will result in a final antibiotic concentration of between 0.1 micrograms per milliliter and 0.075 micrograms per milliliter. When this plate is used, the final concentration of the antibiotic is well below the minimum inhibitory concentration and the organism should grow in normal fashion. Thus, while the large plate need not be used in each instance, it should be used when certain fastidious organisms-antibiotic combinations are suspected, such as *S. aureus*-cephalothin.

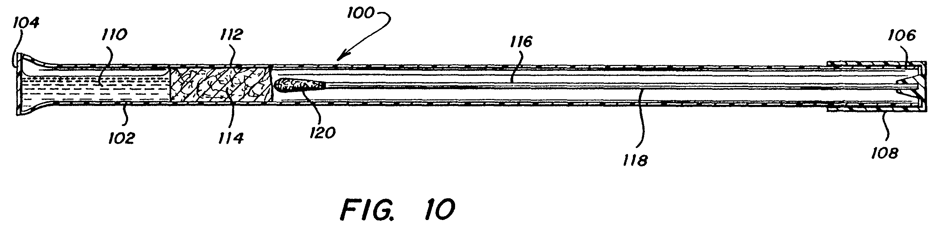

Now referring to Figure 10, another embodiment of the subject invention is depicted which comprises a device for collecting and transporting body secretion samples. Device 100 comprises an elongated flexible tube 102 enclosed at one end 104 and open at its opposite end 106. Cap 108 encloses the open end. Contained within the tube near closed end 104 is a crushable ampule 110 containing a suitable liquid growth media for microbial pathogens. Disposed adjacent ampule 110 is sorbent material 112 which can be any suitable sorbent such as cotton. Sorbent material 112 contains dispersed therein antimicrobial factor deactivation solids 114. Disposed within the open end of tube 106 is swab member 116 which comprises a handle 118 and an absorbent tip 120 for recovering body secretions from a lesion, for example.

Antimicrobial factor deactivation solids 114 are the same material disclosed for use in the lysis-centrifugation vessel described above and can be present in the same relative quantities based upon the amount of solids 114 and growth media 110 and body secretion collected on absorbent tip 120 as the components described above in relation to a blood sample. In operation, cap 106 is removed and swab 116 is removed from the interior of tube 102. The swab contacts body secretion from an open lesion, for example, and is inserted again within the interior of tube 102 and cap 108 is placed over the open end 106 thereof. Thereafter, the portion of tube 102 adjacent closed end 104 is squeezed and ampule 110 is ruptured to cause the liquid growth to be released therefrom and saturate sorbent material 112 and solubilize antimicrobial factor deactivation solids 114. The resulting liquid containing the dissolved antimicrobial factor deactivation system is sorbed by the tip of swab 120 and provides a media for sustaining antimicrobial pathogens present on the tip and also an antimicrobial factor deactivation system for deactivating antimicrobial factors which might be present in the body secretions sorbed on the tip 120 of swab 116. The swab 116 is later removed from container 102 and microbiologically analyzed in a manner described above.

Examples

The following examples are given to better facilitate the understanding of this invention and are not intended to limit the scope thereof. In all Examples:

CFU=Colony-forming units of a microorganism/ml of blood initially inoculated into the tube. Seven and one-half ml of blood are processed per tube.

% Recovery=Percentage of organism recovered in the gradient of all organisms found after processing.

S-Factor=Survival index=Number of CFU recovered from all contents in tube/number of CFU introduced:

S=1 means no kill; S=0.1 means 10% survival.

Example 1

To illustrate the action of sodium polyanetholsulfonate (SPS) on the antibiotic gentamicin. Tests were made comprising the original centrifugation article disclosed and claimed in U.S. Patent 4,212,948. In the original version, each tube contains 0.3 ml of Fluorinert FC48 as cushioning agent and as a blood treating fluid 0.5 milliliters of distilled water containing 0.005 milliliters PPG, 0.008 grams of EDTA and 0.0048 grams of SPS together with 0.018 grams of purified saponin as a lysing agent. The tube was sterile with the aqueous solution having a pH of 7.4 and sufficient vacuum to draw approximately 7.5 milliliters of human blood. A second type tube was prepared except sodium polyanetholsulfonate was added to the aqueous solution in an amount to equal 0.6% by weight of the final concentration of treating fluid and blood sample. Next a series of the above described original tubes and the original tubes plus the sodium polyanetholsulfonate were tested by adding known quantities of *staphylococcus aureus* in a blood sample containing 6 micrograms per milliliter of gentamicin. Blood was lysed, the tubes were held at room temperature (approximately 22°C (72°F)) for 2 hours prior to centrifugation to simulate clinical conditions. Thereafter the tubes were centrifuged as described above and the concentrated material plated on growth media and tested for recovery. The results are set forth below.

TABLE 1
*Staphylococcus aureus* (ATCC 259237)
Gentamicin (6 µg/ml)

| System | CFU | % Recovery | S Factor |
|---|---|---|---|
| Original | 133 | 100 | .06 |
| Original+0.6% SPS | 203 | 80 | .9 |

The original tube gave an overall recovery of 6% while the SPS system gave a recovery of 72% (11.0 fold improvement).

Example 2
A series of original lysis-centrifugation devices were assembled as described in Example 1. A second series of lysis-centrifugation devices were assembled as in Example 1 with the exception that the aqueous phase was modified as follows:
0.5 milliliters of distilled water containing 0.005 milliliters of polypropylene glycol was placed into the tubes.
A total of 0.17 grams of powdered mixture was then added to each tube. The mixture contained the following components:
1.8 grams of purified saponin, 4.8 grams of sodium polyanetholsulfonate, 0.8 grams of thioglycolate, and 9.6 grams of cysteine.
The tubes were sterilized by autoclaving and had a final pH of between 6.6 and 6.8.
Sufficient vacuum was placed in the tube to draw 7.5 milliliters of blood.
In each instance, the stated amount of specific microorganism as illustrated in tables below and antibiotic was added to 7.5 milliliters of blood. The blood was then deposited into the lysis-centrifugation tube and the tube was held at room temperature for 2 hours to simulate clinical conditions, and thereafter was subjected to centrifugation as described in this specification. The concentrated residue in each tube was then plated in equal aliquots on five agar plates containing appropriate growth media which had dimensions of 100 milliliters×20 milliliters and growth was observed. One milliliter of the supervalent remaining after centrifugation was also plated on the five plates. The results are set forth in Table 6 below:

### TABLE 6
#### I. *Staphylococcus aureus* (ATCC #25923)

| Antibiotics*** | Original small plates** | | | Improved small plates | | |
|---|---|---|---|---|---|---|
| | CFU | % Recovery | S Factor | CFU | % Recovery | S Factor |
| No drug | 75 | 91 | .9 ± .2 | 745 | 99 | 1.1± .2 |
| Gentamicin (6 µg) | 133 | 100 | .1 ± .04 | 852 | 99 | 1.0± .1 |
| Tobramycin (4 µg) | 155 | 99 | .4 ± .2 | 259 | 95 | .8± .2 |
| Amikacin (21 µg) | 76 | 99 | 1.0 ± .3 | 152 | 99 | 1.1± .2 |
| Penicillin (20 µg) | 546 | 68 | .01 ± .01 | 305 | 96 | .9± .2 |
| Ampicillin (21 µg) | 403 | 84 | .003±.003 | 319 | 94 | 1.0± .1 |
| Cephalothin (20 µg) | 214 | 100 | .1 ± .03 | 1177 | 76 | .3± .3 |
| Cefoxitin (25 µg) | 158 | 98 | .5 ± .1 | 991 | 99 | .6± .2 |
| Chloramphenicol (18 µg) | 476 | 99 | .6 ± .2 | 495 | 99.8 | 1.1± .3 |
| Tetracycline (9 µg) | 218 | 100 | .5 ± .4 | 584 | 100 | 1.2± .1 |
| Erythromycin (8 µg) | 512 | 100 | .1 ± .05 | 623 | 72 | .8± .2 |
| Gantrisin (100 µg) | 642 | 93 | 1.0 ± .2 | 441 | 99.7 | 1.0± .1 |
| Clindamycin (5 µg) | 180 | 99.7 | 1.2 ± .9 | 123 | 78 | 1.3± .4 |
| Methicillin (9 µg) | 588 | 99.8 | .9 ± .1 | 408 | 92 | 1.1± .3 |
| Vancomycin (8 µg) | 286 | 98 | .6 ± .1 | 1190 | 52 | .8± .2 |
| Piperacillin (60 µg) | 350 | 82 | .005±.007 | 396 | 99 | 1.2± .1 |
| Moxalactam (100 µg) | 676 | 100 | .2 ±.03 | 2345 | 49 | 1.2± .4 |
| Carbenicillin (71 µg/ml) | 483 | 100 | .03 ± .02 | 438 | 99.8 | 1.1± .3 |
| Cefotaxime (20 µg/ml) | 472 | 99 | .3 ± .1 | 595 | 98 | .5± .2 |
| Ticarcillin (150 µg/ml) | 606 | 50 | .01 ± .01 | 553 | 98 | .9± .1 |

# EP 0 107 070 B1

TABLE 6 (contd.)
II. *Escherichia coli* (ATCC #25922)

| Antibiotics*** | Original small plates** | | | Improved small plates | | |
|---|---|---|---|---|---|---|
| | CFU | % Recovery | S Factor | CFU | % Recovery | S Factor |
| No drug | 214 | 98 | 1.2 ± .3 | 886 | 95 | 1.1± .2 |
| Gentamicin (6 µg) | 468 | 100 | .02 ± .01 | 167 | 97 | 1.1±1.0 |
| Tobramycin (4 µg) | 129 | 100 | .6 ± .6 | 399 | 99 | .9± .2 |
| Amikacin (21 µg) | 255 | 100 | .05 ± .03 | 420 | 98 | .8± .1 |
| Penicillin (20 µg) | 526 | 99 | 1.4 ± .3 | 206 | 95 | 1.3± .3 |
| Ampicillin (21 µg) | 490 | 100 | .2 ± .1 | 144 | 98 | .9± .2 |
| Cephalothin (20 µg) | 413 | 100 | .04 ± .04 | 353 | 99 | .5± .1 |
| Cefoxitin (25 µg) | 466 | 99 | .3 ± .06 | 148 | 89 | .8± .3 |
| Chloramphenicol (18 µg) | 323 | 99 | .8 ± .1 | 395 | 99.6 | .7± .3 |
| Tetracycline (9 µg) | 368 | 99 | .5 ± .04 | 320 | 98 | 1.1± .2 |
| Erythromycin (8 µg) | 305 | 99 | 1.0 ± .4 | 212 | 97 | 1.1± .3 |
| Gantrisin (100 µg) | 140 | 99 | 1.5 ±1.0 | 282 | 97 | .8± .3 |
| Ticarcillin (150 µg/ml) | 574 | 99.8 | .5 ± .4 | 651 | 99 | 1.3± .7 |
| Carbenicillin (20 µg/ml) | 1694 | 99.6 | .2 ± .1 | 417 | 98 | 1.0± .2 |
| Piperacillin (60 µg) | 364 | 100 | .7 ± .4 | 1084 | 96 | .9± .2 |
| Cefamandole (20 µg/ml) | 167 | 99.5 | .4 ± .2 | 153 | 100 | .7± .4 |
| Kanamycin (14 µg/ml) | 203 | 100 | .1 ± .03 | 176 | 95 | .3± .1 |
| Methicillin (9 µg/ml) | 198 | 99.8 | 1.1 ± .2 | 146 | 99 | 1.1± .3 |

*All tubes were held at room temperature (20°C) for two hours prior to processing.
**The small plates contained 20 ml of agar media and the large plates contained 80 ml, respectively.
***Numbers in parenthesis represent final concentration of antibiotic used/ml of blood.

The above experiments were repeated except instead of the 100 milliliter by 20 milliliter petri plates containing media, the concentrated residue from each tube was plated on a 150 milliliter by 20 milliliter petri plate which contains between 60 milliliters and 80 milliliters of media and had approximately 2.25 times the surface area of 100 milliliter by 20 milliliter petri plate described which were used to generate the data in Table 6 above. The results are set forth in Table 7 below.

13

# EP 0 107 070 B1

TABLE 7

I. *Staphylococcus aureus* (ATCC #25923)

| Antibiotics*** | Original large plates** | | | Improved large plates | | |
|---|---|---|---|---|---|---|
| | CFU | % Recovery | S Factor | CFU | % Recovery | S Factor |
| No drug | + | + | + | + | + | + |
| Gentamicin (6 μg) | + | + | + | 630 | 92 | .7±.2 |
| Tobramycin (4 μg) | + | + | + | + | + | + |
| Amikacin (21 μg) | + | + | + | + | + | + |
| Penicillin (20 μg) | + | + | + | + | + | + |
| Ampicillin (21 μg) | + | + | + | + | + | + |
| Cephalothin (20 μg) | 214 | 100 | .4 ±.2 | 777 | 100 | .8±.1 |
| Cefoxitin (25 μg) | 467 | 96 | .7 ±.2 | 301 | 99.8 | .9±.1 |
| Chloramphenicol (18 μg) | + | + | + | + | + | + |
| Tetracycline (9 μg) | 266 | 100 | .3 ±.3 | 581 | 99 | .8±.1 |
| Erythromycin (8 μg) | 512 | 100 | .9 ±.2 | 375 | 100 | .6±.2 |
| Gantrisin (100 μg) | + | + | + | + | + | + |
| Clindamycin (5 μg) | + | + | + | + | + | + |
| Methicilin (9 μg) | + | + | + | + | + | + |
| Vancomycin (8 μg) | + | + | + | + | + | + |
| Piperacillin (60 μg) | + | + | + | + | + | + |
| Moxalactam (100 μg) | + | + | + | + | + | + |
| Cefotaxime (20 μg/ml) | 564 | 100 | .8 ±.1 | 567 | 100 | .9±.1 |

14

TABLE 7 (contd.)

II. *Escherichia coli* (ATCC #25922)

| Antibiotics*** | Original large plates** | | | Improved large plates | | |
|---|---|---|---|---|---|---|
| | CFU | % Recovery | S Factor | CFU | % Recovery | S Factor |
| No drug | 237 | 91 | 1.3 ±.3 | 172 | 94 | 1.5±.6 |
| Gentamicin (6 μg) | + | + | + | + | + | + |
| Tobramycin (4 μg) | + | + | + | + | + | + |
| Amikacin (21 μg) | + | + | + | + | + | + |
| Penicillin (20 μg) | + | + | + | + | + | + |
| Ampicillin (21 μg) | + | + | + | + | + | + |
| Cephalothin (20 μg) | 102 | 100 | .1 ±.09 | 288 | 92 | .8±.1 |
| Cefoxitin (25 μg) | 454 | 100 | .5 ±.1 | 406 | 99 | .9±.3 |
| Chloramphenicol (18 μg) | 217 | 99.5 | 1.0±.3 | 420 | 96 | 1.1±.4 |
| Tetracycline (9 μg) | + | + | + | + | + | + |
| Erythromycin (8 μg) | 305 | 100 | .9 ±.2 | 212 | 99 | 1.0±.2 |
| Gantrisin (100 μg) | 140 | 99.8 | 1.4 ±.6 | 231 | 99 | 1.0±.1 |
| Ticarcillin (150 μg/ml) | + | + | + | + | + | + |
| Carbenicillin (20 μg/ml) | + | + | + | + | + | + |
| Piperacillin (60 μg) | + | + | + | + | + | + |
| Cefamandole (20 μg/ml) | 167 | 99 | .5 ±.2 | 209 | 98 | .8±.1 |
| Kanamycin (14 μg/ml) | 330 | 100 | .05±.03 | 352 | 99.6 | .5±.2 |

*All tubes were held at room temperature (20°C) for two hours prior to processing.
**The small plates contained 20 ml of agar media and the large plates contained 80 ml, respectively.
***Numbers in parenthesis represent final concentration of antibiotic used/ml of blood.
+Not tested because recovery is good on small plates.

The data in Tables 6 and 7 illustrate the following important aspects of the invention, namely:
1. In the presence of average serum levels of different antibiotics the original system can lose up to 99.7% of the original inoculum (*Staphylococcus aureus* with ampicillin). For *S. aureus* 13 of 19 antibiotics killed 50% or more of organism within two hours. For *Escherichia coli* this excessive cidal action occurred with nine of the antibiotics.
2. With the new system, the highest kill rate was 70% and a reduction of the inoculum to 50% or less occurred with two antibiotics for *S. aureus* and two with *E. coli*. By adding large plates to the new device, the cidal effect observed in these four cases can be virtually eliminated (S=.8 versus .3; S=.9 versus .5; S=.8 versus .5 and S=.5 versus .3).
In summary, the new system in conjunction with the use of large petri plates is capable of effectively blocking the cidal action of blood and therapeutic antibiotics upon the bacteria present in a blood sample during transport and processing.
The data presented in Tables 8 through 14 below confirm the general effectiveness of this invention on other pathogens commonly isolated from the blood of patients suffering from septicemia.
The procedure set forth in Example 5 above was repeated with eight different potential pathogens and various antibiotics and concentrated residue from each tube was plated on both small and large plates as illustrated in the tables below.

TABLE 8
Enterobacter Cloacae
#1344-2—Small plates

| | % Recovery | | S-Factor | |
|---|---|---|---|---|
| | Old | New | Old | New |
| Ampicillin | 99.5 | 95 | 1.5 ±.5 | .8±.1 |
| Carbenicillin | 0 | 94 | 0 | .6±.3 |
| Ticarcillin | 100 | 86 | .06±.07 | 1.3±.6 |
| Tobramycin | 100 | 75 | .03±.01 | .9±.2 |
| Chloramphenicol | 98 | 80 | .9 ±.2 | 1.2±.3 |
| Tetracycline | 98 | 88 | .9 ±.5 | 1.4±.7 |
| Gantrisin | 97 | 99 | .6 ±.1 | .8±.1 |
| No drug | 97 | 98 | 1.4 ±.2 | 1.2±.1 |
| 'Cefoxitin | 95 | 97 | .9 ±.2 | .8±.2 |
| Cephalothin | 99.6 | 88 | 1.0 ±.2 | .9±.1 |
| Gentamicin | 95 | 99 | .04±.03 | 1.1±.1 |

Large plates

| | % Recovery | | S-Factor | |
|---|---|---|---|---|
| | Old | New | Old | New |
| Tetracycline | 97 | 96 | .7±.1 | .9±.2 |
| Tobramycin | 98 | 93 | .9±.5 | 1.1±.2 |
| Chloramphenicol | + | 97 | + | .9±.3 |

+Not tested because recovery is good on small plates.

**EP 0 107 070 B1**

TABLE 9
Klebsiella Pneumoniae
#632-2—Small plates

|  | % Recovery | | S-Factor | |
| --- | --- | --- | --- | --- |
|  | Old | New | Old | New |
| Ampicillin | 97 | 93 | .5 ±.3 | 1.0±.1 |
| Carbenicillin | 93 | 94 | .1 ±.1 | .8±.2 |
| Ticarcillin | 99 | 89 | 1.0 ±.1 | .9±.1 |
| Tobramycin | 85 | 93 | .3 ±.3 | 1.1±.2 |
| Chloramphenicol | 99 | 93 | 1.3 ±.2 | 1.0±.4 |
| Tetracycline | 98 | 95 | 1.0 ±.1 | .9±.2 |
| Gantrisin | 95 | 98 | 1.0 ±.1 | .9±.1 |
| Cefoxitin | 49 | 97 | .02±.02 | 1.0±.1 |
| No drug | 92 | 93 | 1.1 ±.3 | .7±.1 |
| Cephalothin | 100 | 98 | .2 ±.1 | .5±.2 |
| Gentamicin | 90 | 99 | .02±.01 | .9±.2 |

Large plates

|  | % Recovery | | S-Factor | |
| --- | --- | --- | --- | --- |
|  | Old | New | Old | New |
| Carbenicillin | 92 | 88 | .5±.3 | .7±.2 |

17

TABLE 10
Pseudomonas Aeruginosa
#27853—Small plates

| | % Recovery | | S-Factor | |
| --- | --- | --- | --- | --- |
| | Old | New | Old | New |
| Ampicillin | 97 | 94 | .6±.4 | .8±.2 |
| Carbenicillin | 98 | 95 | .9±.3 | .9±.1 |
| Ticarcillin | 93 | 91 | .3±.1 | 1.2±.1 |
| Tobramycin | 98 | 90 | 1.0±.1 | .9±.2 |
| Chloramphenicol | 96 | 86 | .7±.2 | 1.1±.2 |
| Tetracycline | 95 | 89 | 1.0±.1 | 1.2±.1 |
| Gantrisin | 99 | 98 | 1.2±.2 | .9±.1 |
| No drug | 97 | 97 | 1.6±.3 | .9±.2 |
| Cefotaxime | 99 | 96 | .9±.2 | 1.1±.3 |
| Cefoxitin | 97 | 86 | 1.4±.2 | .9±.4 |
| Cephalothin | 90 | 92 | 1.4±.1 | 1.4±.01 |
| Gentamicin | 98 | 56 | 1.0±.4 | 1.2±.2 |
| Moxalactam | 97 | 87 | .6±.1 | .9±.1 |

# EP 0 107 070 B1

### TABLE 11
### Streptococcus Pneumoniae
#6301—Small plates

|  | % Recovery | | S-Factor | |
| --- | --- | --- | --- | --- |
|  | Old | New | Old | New |
| Penicillin | 76 | 63 | .02 ±.16 | .8±.2 |
| Ampicillin | 43 | 65 | .01 ±.01 | .6±.2 |
| Methicillin | 83 | 86 | .003±.004 | .4±.2 |
| Tobramycin | 97 | 88 | .8 ±.4 | .6±.4 |
| Chloramphenicol | 99 | 93 | .6 ±.4 | .8±.2 |
| Tetracycline | 100 | 99 | .3 ±.2 | .4±.2 |
| Erythromycin | *97 | *98 | *.3 ±.3 | *1.3±.3 |
| Cefoxitin | 97 | 99 | .4 ±.1 | .5±.2 |
| No drug | 93 | 90 | 1.0 ±.03 | 1.0±.1 |
| Gentamicin | 99.8 | 100 | 1.0 ±.1 | 1.1±.1 |

*Incubation period—48 hours.

### Large plates

|  | % Recovery | | S-Factor | |
| --- | --- | --- | --- | --- |
|  | Old | New | Old | New |
| Tetracycline | 99 | 100 | .5±.2 | .7±.2 |
| Tobramycin | 98 | 99 | 1.0±.3 | 1.1±.2 |
| Ampicillin | + | 82 | + | .9±.3 |
| Cefoxitin | 100 | 99 | .2±.1 | .8±.1 |
| Methicillin | + | 97 | + | .6±.1 |
| Penicillin | + | 63 | ± | .9±.1 |

+Not tested because recovery is good on small plates.

TABLE 12
Streptococcus pyogenes
#19615—Small plates

| | % Recovery | | S-Factor | |
| --- | --- | --- | --- | --- |
| | Old | New | Old | New |
| Penicillin | 0.2 | 100 | .02 ±.02 | .6 ±.2 |
| Ampicillin | 0 | 99 | .0002±.0003 | .6 ±.1 |
| Methicillin | 95 | 90 | .2 ±.1 | .8 ±.2 |
| Tobramycin | 98 | 100 | .6 ±.1 | .5 ±.2 |
| Chloramphenicol | 98 | 97 | .5 ±.1 | .4 ±.2 |
| Tetracycline | 100 | 96 | .3 ±.1 | .1 ±.1 |
| Erythromycin | 100 | 100 | .02 ±.01 | .02±.01 |
| Cefoxitin | 98 | 100 | .3 ±.1 | .2 ±.03 |
| No drug | 92 | 95 | 1.0 ±.5 | .5 ±.1 |
| Gentamicin | 99 | 94 | .6 ±.1 | .9 ±.1 |

Large plates

| | % Recovery | | S-Factor | |
| --- | --- | --- | --- | --- |
| | Old | New | Old | New |
| Methicillin | 99 | 99.8 | .6±.1 | 1.7±.2 |
| Tobramycin | 99 | 100 | 1.0±.1 | 1.1±.3 |
| Chloramphenicol | 99 | 99 | .8±.3 | .9±.3 |
| Tetracycline | 99.6 | 100 | .6±.1 | .7±.3 |
| Erythromycin | 100 | 100 | .1±.1 | .1±.1 |
| Cefoxitin | 98 | 95 | .6±.1 | .7±.2 |
| No drug | 98 | 97 | .8±.1 | 1.0±.2 |
| Ampicillin | + | 100 | + | 1.5+.2 |
| Gentamicin | + | 99.5 | + | 1.2+.3 |

TABLE 13
Haemophilus Influenzae
#19418—Small plates

| | % Recovery | | S-Factor | |
|---|---|---|---|---|
| | Old | New | Old | New |
| No drug | 89 | 78 | .6 ±.2 | .9±.4 |
| Ampicillin | 33 | 95 | .01±.01 | .9±.1 |
| Cefoxitin | 80 | 97 | .1 ±.1 | .7±.2 |
| Clindamycin | 96 | 99 | 1.2 ±.2 | 1.1±.2 |
| Erythromycin | 94 | 100 | .4 ±.1 | .7±.2 |
| Gentamicin | 90 | 77 | .4 ±.1 | 1.3±.4 |
| Kanamycin | 94 | 99 | .8 ±.1 | .9±.1 |
| Methicillin | 94 | 99 | .9 ±.2 | .8±.1 |
| Penicillin | 73 | 99 | .1 ±.1 | .6±.1 |
| Tetracycline | 100 | 99 | .2 ±.1 | .6±.1 |
| Vancomycin | 95 | 99 | .7 ±.2 | .8±.2 |

Large plates

| | % Recovery | | S-Factor | |
|---|---|---|---|---|
| | Old | New | Old | New |
| No drug | 95 | 99 | .9±.1 | 1.2±.1 |
| Cefoxitin | 93 | 95 | .8±.6 | .7±.2 |
| Gantrisin | 92 | 98 | 1.2±.7 | .6±.1 |
| Penicillin | 100 | 99 | .3±.2 | .6±.2 |

TABLE 14
Bacteroides Fragilis
#23745—Small plates

| | % Recovery | | S-Factor | |
|---|---|---|---|---|
| | Old | New | Old | New |
| No drug | 88 | 51 | .7 ±.2 | 1.0±.3 |
| Carbenicillin | 96 | 82 | .09±.05 | .5±.1 |
| Cefotaxime | 97 | 100 | .7 ±.2 | .8±.1 |
| Cefoxitin | 94 | 99 | .5 ±.3 | 1.1±.5 |
| Chloramphenicol | 87 | 88 | .9 ±.2 | .9±.1 |
| Erythromycin | 98 | 64 | .7 ±.5 | .6±.2 |
| Penicillin | 87 | 51 | .7 ±.1 | .7±.1 |
| Tetracycline | 90 | 90 | .5 ±.1 | .5±.1 |
| Vancomycin | 95 | 99 | .7 ±.2 | .8±.2 |

Clostridium Sporogenes
#19404—Small plates

| | % Recovery | | S-Factor | |
|---|---|---|---|---|
| | Old | New | Old | New |
| No drug | 97 | 93 | .6 ±.1 | .6±.2 |
| Carbenicillin | 98 | 99 | .3 ±.3 | .8±.3 |
| Cefotaxime | 97 | 98 | .5 ±.2 | .5±.2 |
| Chloramphenicol | 96 | 97 | .7 ±.4 | .6±.4 |
| Clindamycin | 99.7 | 100 | .4 ±.2 | .3±.2 |
| Erythromycin | 96 | 99 | .5 ±.2 | .8±.2 |
| Gantrisin | 93 | 99 | .5 ±.1 | .7±.1 |
| Gentamicin | 98 | 98 | .8 ±.2 | .6±.4 |
| Penicillin | 100 | 96 | .08±.04 | .8±.3 |

Once again a new cocktail protected the microorganisms from the cidal effect of the antibiotics. As expected for those antibiotics which do not exert a cidal effect, both the original tube and the modified tube containing the antimicrobial factor deactivation system yielded the same actual recovery of microorganisms. Furthermore, a review of Examples 5 and 6 illustrate the need for a large dilution (1:267) when dealing with a few specific organism-antibiotic combinations, e.g., *S. aureus* with cephalothin. These data suggest that large dilutions will only be required for a few antibiotics, e.g., cephalothin, tetracycline, erythromycin, and certain organisms, e.g., +cocci. The aminoglycosides, penicillin, ampicillin, and chloramphenicol are completely neutralized by the cocktail while the cephalothins are partially neutralized.

Example 3

A series of the original tubes as described in Example 2 and the tubes containing the antimicrobial factor deactivation system as described in Example 2 were utilized to process blood from patients suspected of having septicemia with confirmed positive blood cultures. In each case, blood from the patient was placed in the original tube and the modified tube containing the antimicrobial factor deactivation system. The tubes were centrifuged and the concentrated residue plated on the small petri plates described in Example 2. The results of the tests are set forth in Table 15 below.

**EP 0 107 070 B1**

TABLE 15

| Culture No. | Organism | Count/ml | | % Of change | Antibiotic in serum at time of draw |
|---|---|---|---|---|---|
| | | Original system | New system | | |
| 1. | Acinetobacter sp. | NG | 1.4 | — | None |
| 2. | Enterobacter agglomerans | 0.7 | 0.7 | 0 | None |
| 3. | Enterobacter cloacae | 0.1 | NG | — | Tobramycin & Cefazolin |
| 4. | Enterobacter cloacae | NG | 0.1 | — | Cefoxitin |
| 5. | Escherichia coli | 0.6 | 2.9 | +383 | Penicillin & Trobramycin |
| 6. | Escherichia coli | 1.1 | .7 | −57 | Penicillin & Trobramycin |
| 7. | Escherichia coli | 13.0 | 92.8 | +614 | None |
| 8. | Escherichia coli | 7.3 | 12.5 | +71 | Ticarcillin & Gentamicin |
| 9. | Escherichia coli | 2.1 | 10.2 | +385 | None |
| 10. | Escherichia coli | NG | 0.1 | — | Mefoxin |
| 11. | Escherichia coli | NG | 0.1 | — | Penicillin & Tobramycin |
| 12. | Flavobacterium | NG | 0.6 | — | — |
| 13. | Histoplasma Capsulatum | 10.5 | 7.0 | −50 | Amphotericin B |
| 14. | Histoplasma Capsulatum | 1.6 | 2.6 | +62 | Amphotericin B |
| 15. | Histoplasma Capsulatum | 13.5 | 14.2 | +05 | Amphotericin B |
| 16. | Klebsiella Oxytoca | 8.8 | 20.6 | +134 | None |
| 17. | Klebsiella Oxytoca | 2.1 | 3.9 | +86 | Gentamicin & Ticarcillin |
| 18. | Klebsiella Pneumoniae | 0.1 | 0.1 | — | Gentamicin |
| 19. | Klebsiella Pneumoniae | 11.9 | 19.3 | +62 | Tobramycin & Carbenicillin |
| 20. | Klebsiella Pneumoniae | 130.8 | 78.1 | −67 | None |
| 21. | Klebsiella Pneumoniae | 163.0 | 182.0 | +12 | Tobramycin & Carbenicillin |
| 22. | Klebsiella Pneumoniae | 0.3 | NG | — | Tobramycin & Carbenicillin |
| 23. | Klebsiella Pneumoniae | 0.3 | 1.0 | +227 | Gentamicin & Ticarcillin |
| 24. | Klebsiella Pneumoniae | 7.3 | 8.4 | +15 | Gentamicin & Ticarcillin |
| 25. | Listeria monocytogenes | 0.4 | 9.5 | +227 | Cefoxitin |
| 26. | Pseudomonas aeruginosa | 131.3 | 83.0 | −58 | Tobramycin & Carbenicillin |
| 27. | Pseudomonas fluorenscens | 0.1 | NG | — | None |
| 28. | Staphylococcus aureus | 5.6 | 12.2 | +118 | Methicillin |

Conclusions:
1. In 68% of the positive samples the new tube yielded more organisms/ml of blood. The difference ranged between a low of 5% and a high of 614% increased count.
2. The new system missed three positives while the original system missed five.
3. In four cases the original system gave a higher count. However, this level is well within expected experimental variability.
4. Thirty-six percent (36%) of the patients were not on antibiotics at the time of blood collection. The new system yielded higher counts in 50% of the cases. The difference ranged from a low of 134% and a high of 614% increase.
5. Seventeen (17) cultures were simultaneously positive at the same time. Two cultures (one Listeria and one Escherichia coli) were positive one day earlier in the new system.

As shown in the table, in 68% of the samples, the modified device containing the antimicrobial factor deactivation system yielded higher counts (which ranged between 5% and 614% increase) than did the original device. In five instances, the original device was negative and the new device positive. Although the majority of samples were positive at the same time, there were two cases in which the new device detected a positive culture one day earlier (*E. coli* and one Listeria specimen). Surprisingly, the new device appears to yield greater counts even when the patient was not on antibiotics (3 patients). This indicates that the new device containing the antimicrobial factor deactivation system more effectively blocked the patient's immune system than did the liquid blood treating solution of the original device.

Example 4

A first series of original lysis-centrifugation devices were assembled as described in Example 1. A second series of lysis-centrifugation devices were assembled the same as the second series of such devices in Example 2. A third series of lysis-centrifugation devices were assembled as follows:

To the article as disclosed in U.S. Patent 4,212,948 were added 0.3 milliliters of Fluorinert FC48 as cushioning agent along with the following compounds in dry particulate powder form:

0.008 grams of thioglycolate;

0.048 grams of sodium polyanetholsulfonate; and

0.018 grams of purified saponin.

The tubes in the third series were evacuated sufficient to draw 8 milliliters of blood. This series of tubes was then heated to 121°C for 30 minutes and then allowed to cool to room temperature.

In each instance, the stated amount of specific microorganisms and antibiotics (if any) as illustrated in Tables 16 through 21 below was added to 7.5 milliliters of blood in the first and second series of tubes and 8 milliliters of blood in the third series of tubes. The blood was then deposited into the respective lysis-centrifugation tube and each tube was subjected to centrifugation as described in this specification. Like quantities of each microbial pathogen-antibiotic combination were plated on both large and small petri plates as described in Example 2. The results are set forth in Tables 16 through 21 below:

## TABLE 16
### *Staphylococcus aureus*

| | Original | | | | New liquid | | | | New powder | | | |
| | Small plate | | Large plate | | Small plate | | Large plate | | Small plate | | Large plate | |
| Antibiotics | % Recovery | S-Factor | % Recovery | S-Factor | % Recovery | S-Factor | % Recovery | S-Factor | % Recovery | S-Factor | % Recovery | S-Factor |
|---|---|---|---|---|---|---|---|---|---|---|---|---|
| No drug | 91 | .88 | * | * | 95 | 1.06 | * | * | 95 | .95 | * | * |
| Ampicillin | 84 | .003 | NT | NT | 99 | 1.10 | NT | NT | 94 | .96 | NT | NT |
| Cefamandole | 100 | .006 | 100 | .099 | 99 | .12 | 100 | .46 | 50 | .03 | 99 | .31 |
| Erythromycin | 100 | .06 | 100 | .85 | 83 | .068 | 100 | .64 | 72 | .80 | * | * |
| Vancomycin | 98 | .58 | * | * | 98 | .75 | * | * | 52 | .76 | * | * |

*Unnecessary to test large plates.
NT=Not tested.

## TABLE 17
### *Escherichia coli*

| | Original | | | | New liquid | | | | New powder | | | |
| | Small plate | | Large plate | | Small plate | | Large plate | | Small plate | | Large plate | |
| Antibiotics | % Recovery | S-Factor | % Recovery | S-Factor | % Recovery | S-Factor | % Recovery | S-Factor | % Recovery | S-Factor | % Recovery | S-Factor |
|---|---|---|---|---|---|---|---|---|---|---|---|---|
| No drug | 98 | 1.17 | * | * | 98 | .78 | 94 | 1.5 | 93 | 1.10 | * | * |
| Cephalothin | 100 | .04 | 100 | .10 | 99 | .07 | 100 | .36 | 95 | .76 | 92 | .83 |
| Moxalactam | 38 | .021 | 75 | .008 | 100 | .10 | * | * | 100 | .017 | 100 | .40 |

*Unnecessary to test large plates.

## TABLE 18
### *Streptococcus pneumoniae*

| Antibiotics | Original | | | | New liquid | | | | New powder | | | |
|---|---|---|---|---|---|---|---|---|---|---|---|---|
| | Small plate | | Large plate | | Small plate | | Large plate | | Small plate | | Large plate | |
| | % Recovery | S-Factor | % Recovery | S-Factor | % Recovery | S-Factor | % Recovery | S-Factor | % Recovery | S-Factor | % Recovery | S-Factor |
| No drug | 92 | .70 | 94 | .81 | 84 | .76 | 95 | 1.00 | 96 | .88 | 69 | .97 |
| Ampicillin | 43 | .0084 | NT | NT | 81 | .52 | 95 | .25 | 65 | .60 | 85 | .88 |
| Cefoxitin | 97 | .39 | 100 | .16 | 99 | .51 | 100 | .27 | 99 | .28 | 99 | .77 |
| Penicillin | 76 | .024 | NT | NT | 87 | .57 | 93 | .43 | 63 | .78 | 63 | .87 |

*Unnecessary to test large plates.
NT=Not tested.

## TABLE 19
### *Enterobacter cloacae*

| Antibiotics | Original | | | | New liquid | | | | New powder | | | |
|---|---|---|---|---|---|---|---|---|---|---|---|---|
| | Small plate | | Large plate | | Small plate | | Large plate | | Small plate | | Large plate | |
| | % Recovery | S-Factor | % Recovery | S-Factor | % Recovery | S-Factor | % Recovery | S-Factor | % Recovery | S-Factor | % Recovery | S-Factor |
| No drug | 97 | 1.42 | * | * | — | — | * | * | 98 | 1.20 | * | * |
| Chloramphenicol | 98 | .91 | * | * | 89 | 1.6 | 97 | .93 | 80 | 1.15 | * | * |
| Tobramycin | 100 | .028 | 98 | .94 | 94 | .70 | 93 | 1.07 | 75 | .85 | * | * |

*Unnecessary to test large plates.
—Discontinued production of liquid tube (dry tube, results only).

EP 0 107 070 B1

## TABLE 20
### *Pseudomonas aeruginosa*

| Antibiotics | Original | | | | New liquid | | | | New powder | | | |
| | Small plate | | Large plate | | Small plate | | Large plate | | Small plate | | Large plate | |
| | % Recovery | S-Factor | % Recovery | S-Factor | % Recovery | S-Factor | % Recovery | S-Factor | % Recovery | S-Factor | % Recovery | S-Factor |
|---|---|---|---|---|---|---|---|---|---|---|---|---|
| Ampicillin | 97 | .56 | * | * | 87 | .84 | * | * | 94 | .83 | * | * |
| Carbenicillin | 97 | .125 | * | * | 92 | .85 | * | * | 56 | .92 | * | * |

*Unnecessary to test large plates.

## TABLE 21
### *Klebsiella pneumoniae*

| Antibiotics | Original | | | | New liquid | | | | New powder | | | |
| | Small plate | | Large plate | | Small plate | | Large plate | | Small plate | | Large plate | |
| | % Recovery | S-Factor | % Recovery | S-Factor | % Recovery | S-Factor | % Recovery | S-Factor | % Recovery | S-Factor | % Recovery | S-Factor |
|---|---|---|---|---|---|---|---|---|---|---|---|---|
| Carbenicillin | 93 | .143 | 92 | .54 | 93 | .66 | 88 | .72 | 94 | .76 | * | * |
| Cefoxitin | 49 | .02 | NT | NT | 72 | .86 | NT | NT | 97 | .98 | NT | NT |

*Unnecessary to test large plates.
NT=Not tested.

As can be seen from the data above, the lysis-centrifugation devices made in accordance with the subject invention that contain the dry particulate powdered antimicrobial factor deactivation system of the subject invention performed at least as well as the systems in accordance with this invention containing the antimicrobial deactivation system in aqueous solution within the tube. Both of the new systems clearly outperform the original system as set forth in the Examples.

Example 5

A first series of original lysis-centrifugation devices were assembled as described in Example 1. A second series of lysis-centrifugation devices were assembled in the same manner as the third series of lysis-centrifugation devices which were used to obtain the data set forth in Tables 16 through 21 of Example 4.

In each instance, the stated amount of specific microorganism as illustrated in Table 22 below was added to 7.5 ml of blood in the first series of tubes and to 8 ml of blood in the second series of tubes. The blood was then deposited in the respective lysis-centrifugation tubes and the tubes were then held at 21°C for the time period set forth in Table 22 below. Each tube was next subjected to centrifugation and the concentrated contents plated on growth media as described in the specification.

As can be seen from the data set forth in Table 22, certain species of bacteria propagate or die in the original tube when held for a period of 24 hours. Surprisingly, these same species did not substantially grow or die in the second series of new tubes containing the antibiotic deactivation agent. While it is not recommended that the centrifugation tubes be held for lengthy periods of time, it has been found in the hospital environment that such tubes are held for time periods before processing. While the data shows no substantial propagation of most specie within the new tube at 24 hours, it is believed that the tubes should be processed as quickly as possible and certainly before a hold time of 12 hours has been completed. Furthermore, to assure against growth of some species of bacteria such as *Enterobacter cloacae*, sodium chloride can be added, such as in the urine examples as set forth in Example 8 below. Sodium chloride can be present in an amount from about 0.1% to about 10% by weight of the final process treating solution and blood sample, and preferably in the range of from about 1% to about 5% and most preferably at about 3%.

TABLE 22

Blood hold time reconstruction checks

| Organism | | CFU | % Recovery | | S-Factor | |
|---|---|---|---|---|---|---|
| | | | 2 HR | 24 HR | 2 HR | 24 HR |
| *Haemophilus influenzae* | original | 501 | 86 | 91 | .8±.2 | .2 ± .1 |
| (19418) | new | 501 | 93 | 76 | .8±.1 | .5 ± .3 |
| *Streptococcus pyogenes* | original | 142 | 99.7 | 89 | .8±.3 | .4 ± .1 |
| (1344-2) | new | 142 | 100 | 100 | .9±.3 | .3 ± .1 |
| *Pseudomonas aeruginosa* | original | 332 | 99 | TNTC | 2.2±.8 | TNTC |
| (27853) | new | 332 | 95 | 94 | 1.2±.4 | 4.4 ±2.6 |
| *Staphylococcus aureus* | original | 552 | 99 | 100 | .8±.03 | .03± .004 |
| (25923) | new | 1317 | 98 | 97 | 1.0±.3 | 1.5 ± .6 |
| *Escherichia coli* | original | 826 | 98 | TNTC | 1.7±.5 | TNTC |
| (25922) | new | 1057 | 100 | 100 | .9±.1 | 1.0 ± .4 |
| *Streptococcus pneumoniae* | original | 602 | 93 | 98 | 1.0±.3 | .8 ± .04 |
| (6301) | new | 360 | 90 | 99 | 1.0±.1 | 1.1 ± .2 |
| *Enterobacter cloacae* | original | 1236 | 98 | — | 1.2±.5 | TNTC |
| (1344-2) | new | 1236 | 99 | — | .9±.2 | TNTC |

TNTC—too numerous to count.

Example 6

A series of lysis-centrifugation devices were assembled the same as the second series of devices containing the antimicrobial factor deactivation system as in Example 5. To each tube was added 8 ml of blood containing 842 CFU of *E. coli* and 20 µg/ml of the antibiotic cefotaxime as well as the stated amount of beta-lactamase enzyme illustrated in Table 22 below. The beta-lactamase enzyme used was beta-lactamase (*Bacillus cereus*), lot No. 203435, order No. 426205, Calbiochem-Behring Corporation, La Jolla, California, NOTE: beta-lactamase I—13 units of activity to beta-lactamase II—1 unit of activity. The blood was then deposited into the respective lysis-centrifugation tubes and each tube was subjected to centrifugation as described in the specification. Like quantities of microbial pathogen-antibiotic-enzyme combination were plated on small petri plates as described in Example 2. The results are set forth in Table 23 below.

TABLE 23

E. coli—Cefotaxime 20 µg/ml

| Units of enzyme | Percent recovery | S-Factor |
|---|---|---|
| 0 | 58 | .02 |
| .01 | 100 | .011 |
| 0.1 | 100 | .052 |
| 1.0 | 99 | .33 |
| 2.0 | 99 | 1.17 |
| 4.0 | 99 | .82 |
| 5.0 | 99.8 | .95 |

As can be seen from Table 23, the beta-lactamase as an integral part of the antimicrobial factor deactivation system will effectively function to block the activity of the antibiotic and prevent killing of the microbial pathogen while contained within the lysis-centrifugation tube.

As a comparison, a second series of tubes were assembled as described in Example 1 and to each tube was added 765 CFU of E. coli, 20 µg/ml of cefotaxime, the units of beta-lactamase enzyme as illustrated in Table 24 and 7.5 ml of blood. The tubes were then centrifuged and samples were cultured as described above, and the results are set forth in Table 24 below.

TABLE 24

| Units of enzyme | Percent recovery | S-Factor |
|---|---|---|
| 0 | 93 | .08 |
| 1 | 90 | .04 |
| 5 | 95 | .155 |

The results of Table 24 when compared with Table 23 indicate that the addition of the enzyme does not satisfactorily improve the S values when used in a lysis-centrifugation tube which does not contain the antimicrobial factor deactivation system.

Further tests were made comparing a first series of lysis-centrifugation tubes identical to those prepared in conjunction with Table 24 above and containing no antimicrobial factor deactivation system; a second series of lysis-centrifugation tubes identical to those used in conjunction with Table 23 above but containing no enzyme; and a third series of lysis-centrifugation tubes which were the same as the second series of tubes but which contained the indicated amounts of beta-lactamase enzyme as set forth in Tables 25 through 30 below. The blood containing between 200 and 1000 CFU of the indicated bacteria was added to each tube and the tubes were processed as described above in this example and the results are set forth in Tables 25 through 30 below:

TABLE 25
*E. coli* 25922

| Antibiotic | µg/ml | First series of tubes | Second series of tubes no enzyme | Third series of tubes with enzyme* units | | | | | | | | |
| --- | --- | --- | --- | --- | --- | --- | --- | --- | --- | --- | --- | --- |
| | | | | .01 | .1 | 1. | 2. | 2.5 | 3. | 4. | 5. | 10. |
| Cephalothin | 20 | .04 | .05 | | | .40 | .90 | | | | 1.30 | |
| Cefamandole | 20 | .35 | .35 | | | 1.0 | .63 | | | | | |
| Cefoxitin | 25 | .27 | .76 | | | .91 | | | 1.76 | | | |
| Cefotaxime | 20 | .08 | .02 | .01 | .05 | .33 | 1.2 | | | .82 | .95 | |
| Moxalactam | 100 | .02 | .01 | | | .03 | .05 | | .004 | | .04 | .002 |
| Moxalactam | 50 | | .03 | | | .04 | | | .05 | | | |
| Moxalactam | 40 | | .01 | | | .10 | | .22 | .12 | | .16 | |
| Moxalactam | 20 | | .33 | | | .20 | | | .16 | | .65 | |
| Moxalactam | 10 | | .84 | | | 1.01 | | | .92 | | | |
| Cefob | | .001 | .003 | | | .84 | | | 1.01 | | | |

## TABLE 26
### *Staph. aureus* 25923

| Antibiotic | µg/ml | First series of tubes | Second series of tubes no enzyme | Third series ADS results with enzyme* units | | | | | | | | |
|---|---|---|---|---|---|---|---|---|---|---|---|---|
| | | | | .01 | .1 | 1. | 2. | 2.5 | 3. | 4. | 5. | 10. |
| Cephalothin | 20 | .08 | .04 | .03 | .002 | .50 | .85 | | | | 1.65 | |
| Cefamandole | 20 | .006 | .03 | | | .81 | | | .62 | | .81 | |
| Cefotaxime | 20 | .28 | .52 | | | .96 | | | 1.02 | | | |
| Cefob | 50 | .009 | .025 | | | .72 | | | .80 | | | |

## TABLE 27
### *Kleb. pneumo.* 632-2

| Antibiotic | µg/ml | First series of tubes | Second series of tubes no enzyme | Third series ADS results with enzyme* units | | | | | | | | |
|---|---|---|---|---|---|---|---|---|---|---|---|---|
| | | | | .01 | .1 | 1. | 2. | 2.5 | 3. | 4. | 5. | 10. |
| Cefotaxime | 20 | 0 | 0 | | .03 | | | | .58 | | | |
| Moxalactam | 20 | — | — | | .23 | | | | .22 | | | |

## TABLE 28
### *Ent. cloacae* 1344-2

| Antibiotic | µg/ml | First series of tubes | Second series of tubes no enzyme | Third series ADS results with enzyme* units | | | | | | | | |
|---|---|---|---|---|---|---|---|---|---|---|---|---|
| | | | | .01 | .1 | 1. | 2. | 2.5 | 3. | 4. | 5. | 10. |
| Cefotaxime | 20 | .12 | .013 | | | .79 | | | .52 | | | |
| Moxalactam | 20 | — | — | | | .12 | | | .26 | | | |

### TABLE 29
#### *Hema. influen.*—19418

| Antibiotic | µg/ml | First series of tubes | Second series of tubes no enzyme | Third series ADS results with enzyme* units | | | | | | | | |
| | | | | .01 | .1 | 1. | 2. | 2.5 | 3. | 4. | 5. | 10. |
|---|---|---|---|---|---|---|---|---|---|---|---|---|
| Cefotaxime | 20 | .003 | .001 | | | .153 | | | .46 | | | |
| Moxalactam | 40 | — | — | | | .005 | | | .004 | | | |

### TABLE 30
#### *Strep. pneumo.*—6301

| Antibiotic | µg/ml | First series of tubes | Second series of tubes no enzyme | Third series ADS results with enzyme* units | | | | | | | | |
| | | | | .01 | .1 | 1. | 2. | 2.5 | 3. | 4. | 5. | 10. |
|---|---|---|---|---|---|---|---|---|---|---|---|---|
| Cefoxitin | 25 | .39 | .28 | | | .33 | | | .18 | | | |
| Cefotaxime | 20 | .001 | 0 | | | .23 | | | .62 | | | |

EP 0 107 070 B1

The data shown in Tables 25—30 show that the addition of the enzyme to the antimicrobial deactivation factor system of the subject invention effectively enhances the neutralization properties thereof for the above indicated class of antibiotics.

Example 7

Lysis-centrifugation tubes containing the antibiotic deactivation system utilized in the second series as set forth in Tables 25 through 30 above were made up. To a first series of these tubes was added the amount of beta-lactamase enzyme set forth in Table 31 below. The tubes were then subjected to cobalt sterilization and thereafter 8 ml of blood containing the microbial pathogen and the antibiotic as set forth in Table 31 were added thereto and processed as set forth in Example 6. A second series of the tubes were steam sterilized and thereafter the indicated amount of beta-lactamase enzyme was added thereto and thereafter the 8 ml of blood with the indicated amount of microbial pathogen and antibiotic was added thereto and the tubes were centrifuged and processed as set forth in Example 6. The results of these tests were set forth in Tables 31 and 32 below.

TABLE 31
Cobalt sterilization

| Units of enzyme | E. coli 25922 cefotaxime 20 μg/ml | | Staph. aureus 25923 cephalothin 20 μg/ml | |
|---|---|---|---|---|
| | Percent recovery | S-Factor | Percent recovery | S-Factor |
| 0.1 | 67 | .01 | 100 | .04 |
| 1.0 | 83 | .06 | 97 | .17 |
| 5.0 | 81 | .41 | 97 | .33 |

TABLE 32
Steam autoclave sterilization

| Units of enzyme | E. coli 25922 cefotaxime 20 μg/ml | | Staph. aureus 25923 cephalothin 20 μg/ml | |
|---|---|---|---|---|
| | Percent recovery | S-Factor | Percent recovery | S-Factor |
| .01 | 100 | .05 | 17 | .002 |
| 1.0 | 99 | .33 | 26 | .50 |
| 5.0 | 99.8 | .95 | 34 | 1.65 |

As can be seen by a comparison of the data in Table 31 with Table 32, the loss of enzyme activity due to cobalt sterilization ranges from 20% to 80%, depending on the concentration of the enzyme. However, Table 31 clearly illustrates that cobalt sterilization can be effectively utilized, and when used, increased amounts of the enzyme should be added to the tube prior to the sterilization. It should be noted that other chemicals are anticipated for use within the antimicrobial factor deactivation system depending somewhat on the type of antimicrobial factors which are anticipated to be present in the sample. For example, other water-soluble compounds which are antagonistic to other classes of antimicrobial substances such as sodium hypochlorite, heavy metals and the like include substances like sodium bisulfite and sulfhydryls, for example. As indicated, the antimicrobial factor deactivation system of the subject invention finds special utility in the lysis-centrifugation tube such as set forth in U.S. 4,131,512 and U.S. 4,212,948. In addition, the antimicrobial factor deactivation system finds utility in the lysis-centrifugation tube as set forth in U.S. 4,164,449. In addition, the antimicrobial factor deactivation system of the subject invention can be utilized in a blood treating tube for neonates which simply will include a standard single stopper vacuum tube designed to draw between 1 and 2 milliliters of blood. The tube would contain no substance other than the antimicrobial factor deactivation system of the subject invention and saponin if desired. The blood can be treated upon injection in the tube and then directly plated upon growth media.

The above Examples illustrate the beneficial effect of the antimicrobial factor deactivation system of the subject invention when used in a lysis-centrifugation tube for analyzing microbial pathogens within blood samples. However, the antimicrobial factor deactivation system of the present invention finds utility in protecting microorganisms in sample fluids other than blood which are collected and later analyzed for the presence of microbial pathogens. For example, the antimicrobial factor deactivation system of the subject invention can protect microorganisms present in swabs, urine, sputum, spinal fluid and other body fluids during transit. It is well known that these fluids also contain both humoral and chemical antimicrobials (if the patient is being treated with antibiotics). With urine samples, the concentration of antibiotics may actually exceed that present in serum. An example of a modified antimicrobial factor deactivation system for neutralizing antibiotics in urine is present in Example 8 below.

Example 8
The following example was performed to test the ability of the antimicrobial factor deactivation system urine cocktail to block conventional therapeutic antibiotics and hold the microbial population, present in the urine, stable for up to 24 hours.
The following dry mixture was placed in each of a series of tubes:
0.03 grams of sodium polyanethosulfonate,
0.005 grams of thioglycolate
0.1 grams of ICN free-base cysteine,
0.1 grams of sodium bicarbonate.
The various antibiotics, listed in Tables 33—38 below, were added, at the concentrations also specified therein, to the tubes containing the above-described antimicrobial factor deactivation urine cocktail and to an equal number of tubes without the urine cocktail. Five milliliters of sterile urine was then added to all tubes after which the tubes were vigorously mixed. Control tubes contained either urine alone or urine plus the above-described antimicrobial factor deactivation urine cocktail. No antibiotics were added to control tubes. The microorganisms listed in Tables 33—38 below were adjusted to a McFarlin of 0.5 and then diluted 1:100 with sterile culture media. A 0.1 milliliter aliquot of a single microorganism was added to each urine containing tube and the resultant mixture vigorously agitated. A ten microliter aliquot from each tube containing the resultant mixture was immediately inoculated on tryptic soy agar plates and spread with a sterile spreader. The inoculated plates were incubated overnight in an environment and temperature appropriate for the microorganism employed. The tubes were then allowed to stand at room temperature for 24 hours. Additional ten microliter aliquots were plated as before herein described at the time intervals indicated in Tables 33—38 below. All plating was done in quadruplicate and the S-Factor recorded as an average of the quadruplicate plating in Tables 33—38 below.

TABLE 33
*Escherichia coli* 25922

| Antibiotic** | Hour time points | | | |
|---|---|---|---|---|
| | 2 | 4 | 6 | 24 |
| *No drug | — | .92 | 1.05 | .84 |
| No drug | — | 2.01 | TNTC | TNTC |
| *Amikacin (210) | — | .73 | .62 | .33 |
| Amikacin (210) | — | 0 | 0 | 0 |
| *Ampicillin (210) | — | .92 | .75 | .70 |
| Ampicillin (210) | — | .02 | .001 | 0 |
| *Carbenicillin (200) | 1.12 | .90 | — | .31 |
| Carbenicillin (200) | .83 | .37 | — | .003 |
| *Cefamandole (200) | .88 | .45 | — | .31 |
| Cefamandole (200) | .37 | .33 | — | .005 |
| *Cefobid (500) | — | 1.16 | 1.63 | 1.68 |
| Cefobid (500) | — | .03 | 0 | 0 |
| *Cefotaxime (200) | — | 1.03 | 1.55 | 1.65 |
| Cefotaxime (200) | — | .009 | 0 | 0 |
| *Cefoxitin (250) | .72 | .33 | — | .17 |
| Cefoxitin (250) | .23 | .05 | — | .002 |
| *Cephalothin (200) | — | .47 | .86 | .53 |
| Cephalothin (200) | — | .01 | .01 | .04 |
| *Chloramphenicol (180) | — | 1.03 | 1.03 | .89 |
| Chloramphenicol (180) | — | 0 | 0 | 0 |
| *Erythromycin (80) | — | .70 | .75 | .59 |
| Erythromycin (80) | — | 1.36 | 1.49 | .42 |
| *Gantrisin (1000) | .70 | .93 | — | .56 |
| Gantrisin (1000) | 0 | 0 | — | 0 |
| *Gentamicin (60) | — | .81 | .38 | .28 |
| Gentamicin (60) | — | 0 | 0 | 0 |
| *Piperacillin (600) | .76 | .67 | — | .33 |
| Piperacillin (600) | .48 | .55 | — | .01 |
| *Tetracycline (90) | 1.12 | 1.16 | — | .56 |
| Tetracycline (90) | .20 | .02 | — | 0 |
| *Tobramycin (40) | — | 1.05 | 1.06 | .90 |
| Tobramycin (40) | — | 0 | 0 | 0 |

—Time point not included in reconstruction.
*Antimicrobial factor deactivation system urine cocktail is present.
**Number in parenthesis represents final concentration (μg/ml) of antibiotic urine.
TNTC=too numerous to count.

TABLE 34

*Klebsiella pneumoniae*

| Antibiotic** | Hour time points | | |
|---|---|---|---|
| | 4 | 6 | 24 |
| *No drug | 1.10 | 1.31 | 1.08 |
| No drug | 1.00 | 3.7 TNTC | TNTC |
| *Amikacin (210) | .86 | .64 | .27 |
| Amikacin (210) | 0 | 0 | 0 |
| *Ampicillin (210) | 1.13 | 1.18 | .67 |
| Ampicillin (210) | 1.30 | TNTC | TNTC |
| *Carbenicillin (710) | 1.06 | .83 | .33 |
| Carbenicillin (710) | .15 | .01 | .03 |
| *Cefamandole (200) | 1.15 | 1.07 | .49 |
| Cefamandole (200) | .16 | .11 | .02 |
| *Cefobid (500) | .67 | .90 | .90 |
| Cefobid (500) | .007 | .007 | 0 |
| *Cefotaxime (200) | 1.00 | 1.33 | 3.83 |
| Cefotaxime (200) | .02 | .008 | .015 |
| *Cefoxitin (250) | .64 | .80 | .49 |
| Cefoxitin (250) | .04 | .05 | .14 |
| *Cephalothin (200) | .70 | .81 | .18 |
| Cephalothin (200) | .10 | .02 | .03 |
| *Chloramphenicol (180) | 1.22 | 1.07 | .59 |
| Chloramphenicol (180) | .84 | .87 | .39 |
| *Erythromycin (80) | 1.27 | 1.00 | 1.08 |
| Erythromycin (80) | 1.63 | 2.25 | TNTC |
| *Gantrisin (1000) | .82 | .67 | 1.00 |
| Gantrisin (1000) | 2.29 | .60 TNTC | 3.8 TNTC |
| *Gentamicin (60) | .94 | .73 | .22 |
| Gentamicin (60) | 0 | 0 | 0 |
| *Moxalactam (1000) | 3.48 | 3.52 | 2.30 |
| Moxalactam (1000) | .11 | 0 | 0 |
| *Piperacillin (600) | .52 | .84 | .46 |
| Piperacillin (600) | 1.07 | .15 | .34 |
| *Tetracycline (90) | .75 | 3.02 | .23 |
| Tetracycline (90) | .90 | .97 | .23 |
| *Tobramycin (40) | .85 | .95 | .53 |
| Tobramycin (40) | 0 | 0 | 0 |

—Time point not included in reconstruction.

*Antimicrobial factor deactivation system urine cocktail is present.

**Number in parenthesis represents final concentration (μg/ml) of antibiotic urine.

TNTC=too numerous to count.

36

TABLE 35

*Pseudomonas aeruginosa*

| Antibiotic** | Hour time points | | |
|---|---|---|---|
| | 4 | 6 | 24 |
| *No drug | .92 | .77 | .58 |
| No drug | 1.48 | 2.49 | TNTC |
| *Amikacin (210) | 1.33 | 1.41 | .47 |
| Amikacin (210) | .58 | .15 | .01 |
| *Carbenicillin (710) | 1.16 | 1.36 | .80 |
| Carbenicillin (710) | .88 | .38 | .08 |
| *Moxalactam (1000) | 1.10 | .86 | .41 |
| Moxalactam (1000) | .52 | .18 | .06 |
| *Piperacillin (600) | 1.54 | 1.05 | .91 |
| Piperacillin (600) | .32 | .98 | .23 |
| *Tobramycin (40) | .90 | .42 | 0.5 |
| Tobramycin (40) | .22 | .05 | 0 |

—Time point not included in reconstruction.
*Antimicrobial factor deactivation system urine cocktail is present.
**Number in parenthesis represents final concentration (µg/ml) of antibiotic urine.
TNTC=too numerous to count.

TABLE 36

*Proteus vulgaris*

| Antibiotic** | Hour time points | | |
|---|---|---|---|
| | 4 | 6 | 24 |
| *No drug | .57 | 1.07 | 3.11 |
| No drug | .88 | 1.04 | TNTC |
| *Amikacin (210) | .72 | .92 | 1.67-swarm |
| Amikacin (210) | .15 | 0 | 0 |
| *Cefamandole (200) | .50 | .50 | .34 |
| Cefamandole (200) | .20 | .21 | .007 |
| *Piperacillin (600) | 2.17 | 1.80 | 3.7-? |
| Piperacillin (600) | 0 | 0 | 0 |
| *Tobramycin (40) | .67 | .92 | swarm |
| Tobramycin (40) | .18 | .03 | 0 |

—Time point not included in reconstruction.
*Antimicrobial factor deactivation system urine cocktail is present.
**Number in parenthesis represents final concentration (µg/ml) of antibiotic urine.
TNTC=too numerous to count.

## EP 0 107 070 B1

### TABLE 37
*Enterobacter cloacae*

| Antibiotic** | Hour time points | | |
|---|---|---|---|
| | 4 | 6 | 24 |
| *No drug | 1.0 | .77 | 2.47 |
| No drug | 2.21 | 7.91 | TNTC |
| *Amikacin (210) | 1.12 | .62 | .11 |
| Amikacin (210) | 0 | 0 | 0 |
| *Ampicillin (210) | .99 | 1.68 | 3.60 |
| Ampicillin (210) | .18 | .07 | .02 |
| *Carbenicillin (710) | .76 | .76 | .14 |
| Carbenicillin (710) | .25 | .23 | .20 |
| *Cefamandole (200) | .89 | 1.44 | 1.11 |
| Cefamandole (200) | 1.13 | .48 | .44 |
| *Cefobid (500) | .07 | .13 | .04 |
| Cefobid (500) | .03 | .003 | .0005 |
| *Cefotaxime (200) | .92 | 1.04 | .59 |
| Cefotaxime (200) | .12 | .02 | .05 |
| *Cefoxitin (250) | 1.01 | 1.14 | 2.1 |
| Cefoxitin (250) | .25 | .52 | 4.34 |
| *Cephalothin (200) | .88 | 1.03 | .49 |
| Cephalothin (200) | 2.41 | 4.37 | TNTC |
| *Chloramphenicol (180) | .97 | .94 | .94 |
| Chloramphenicol (180) | 1.06 | .97 | .76 |
| *Erythromycin (80) | .95 | 1.04 | 1.06 |
| Erythromycin (80) | 1.23 | 1.27 | TNTC |
| *Gantrisin (1000) | .78 | 1.01 | .99 |
| Gantrisin (1000) | 1.74 | 4.02 | 1.04-TNTC |
| *Gentamicin (60) | .68 | .55 | .13 |
| Gentamicin (60) | 0 | 0 | 0 |
| *Moxalactam (1000) | 5.28 | 5.68 | 5.80 |
| Moxalactam (1000) | .10 | 0 | 0 |
| *Piperacillin (600) | .91 | .88 | 1.84 |
| Piperacillin (600) | .65 | .24 | .09 |
| *Tetracycline (90) | .85 | 1.73 | 1.23 |
| Tetracycline (90) | 1.03 | 2.43 | .58 |
| *Tobramycin (40) | .92 | .81 | 2.12 |
| Tobramycin (40) | 0 | 0 | 0 |

—Time point not included in reconstruction.
*Antimicrobial factor deactivation system urine cocktail is present.
**Number in parenthesis represents final concentration (µg/ml) of antibiotic urine.
TNTC=too numerous to count.

38

TABLE 38

*Staphylococcus aureus*

*ADS urine
Regular urine

| | Hour time points | | | | |
|---|---|---|---|---|---|
| | 0 | 2 | 4 | 6 | 24 |
| *No drug | 1.00 | — | .91 | 1.05 | 1.20 |
| No drug | 1.00 | — | .98 | 1.06 | TNTC |
| *Amikacin (210) | 1.00 | — | .73 | .37 | .33 |
| Amikacin (210) | 1.00 | — | .071 | .008 | .001 |
| *Ampicillin (210) | 1.00 | .68 | .70 | — | .63 |
| Ampicillin (210) | 1.00 | 1.57 | 1.52 | — | .86 |
| *Carbenicillin (710) | 1.00 | .58 | .57 | — | .43 |
| Carbenicillin (710) | 1.00 | .70 | .80 | — | .37 |
| *Cefamandole (200) | 1.00 | .78 | .68 | — | .57 |
| Cefamandole (200) | 1.00 | .70 | .63 | — | .14 |
| *Cefobid (500) | 1.00 | — | 1.15 | 1.15 | 1.17 |
| Cefobid (500) | 1.00 | — | .58 | .42 | .08 |
| *Cefotaxime (200) | 1.00 | — | .83 | .82 | 1.00 |
| Cefotaxime (200) | 1.00 | — | 1.18 | .82 | .26 |
| *Cefoxitin (250) | 1.00 | .79 | .9 | — | .76 |
| Cefoxitin (250) | 1.00 | .79 | .57 | — | .21 |
| *Cephalothin (200) | 1.00 | — | .87 | 1.2 | 2.03 |
| Cephalothin (200) | 1.00 | — | .74 | 1.03 | .39 |
| *Chloramphenicol (180) | 1.00 | — | .73 | .80 | .63 |
| Chloramphenicol (180) | 1.00 | — | .41 | .28 | .086 |
| *Erythromycin (80) | 1.00 | — | .81 | .85 | .78 |
| Erythromycin (80) | 1.00 | — | .23 | .090 | .012 |
| *Gantrisin (1000) | 1.00 | — | .79 | .84 | .66 |
| Gantrisin (1000) | 1.00 | — | .89 | .21 | 2.22 |
| *Gentamicin (60) | 1.00 | — | .71 | .81 | .36 |
| Gentamicin (60) | 1.00 | — | 0 | 0 | 0 |
| *Moxalactam (1000) | 1.00 | — | .8 | .76 | .62 |
| Moxalactam (1000) | 1.00 | — | .86 | .65 | .26 |
| *Piperacillin (600) | 1.00 | .45 | .58 | — | .38 |
| Piperacillin (600) | 1.00 | .72 | .47 | — | .19 |
| *Tetracycline (90) | 1.00 | .64 | .73 | — | .73 |
| Tetracycline (90) | 1.00 | .067 | .012 | — | 0 |
| *Tobramycin (40) | 1.00 | — | 1.02 | .99 | .93 |
| Tobramycin (40) | 1.00 | — | .093 | .013 | 0 |

—Time point not included in reconstruction.
*Antimicrobial factor deactivation system urine cocktail is present.
**Number in parenthesis represents final concentration (μg/ml) of antibiotic urine.
TNTC=too numerous to count.

Tables 33—38 clearly demonstrate the ability of the antimicrobial factor deactivation urine cocktail to block conventional therapeutic antimicrobials, antibiotics, in the urine and to hold the microbial count relatively constant in the absence of antimicrobials.

It should be noted that with normal urine minus antibiotics the common pathogenic organisms will grow (*E. coli, K. pneumoniae, P. aeruginosa, P. vulgaris,* and *E. cloacae*) over a 24 hour period at room temperature. Hence, if the urine specimen is not analyzed promptly, it can lead to a false positive result. In the presence of average urine concentrations of antibiotics (10× that of blood serum) sensitive pathogenic organisms rapidly die. This could lead the laboratory to the conclusion that the specimen does not contain a significant number of pathogenic organisms ($10^5$) when in reality the specimen did contain the pathogens at this level at the time of collection. In other words, if two or more hours have elapsed between collection and laboratory processing, the count obtained may be as low as $10^3$, i.e., considered not significant.

The urine antimicrobial factor deactivation system achieves two major improvements, namely:

1. It is capable of effectively blocking the cidal effects of antibiotics for at least 6 hours, and in the most of cases, for up to 24 hours.

2. The number of organisms present at time zero in the presence or absence of antibiotics remains constant for up to at least 6 hours.

In conclusion, the unique features of this urine antimicrobial factor deactivation system allows the urine specimen to be held for up to 24 hours prior to processing with no deleterious effect on the microbial integrity of the sample. Refrigeration is not required, and the system is effective in the absence or presence of antimicrobials.

As can be seen from the above Examples, the antimicrobial factor deactivation system which falls within the scope of the subject invention has many uses. The ability of the antimicrobial factor deactivation system to hold the microbial count constant may allow for detection of significant microbial species which would otherwise be masked by the overgrowth of more rapidly dividing organisms.

Example 9

The following dry mixture was placed in each of a series of tubes:

0.03 grams of sodium polyanethosulfonate,

0.005 grams of thioglycolate,

0.1 grams of ICN free-base cysteine,

0.1 grams of sodium bicarbonate.

Various percentages, by weight thereof, of sodium chloride, indicated in Table 38 below, were then added to individual tubes containing the above-described antimicrobial factor deactivation system urine cocktail. A five milliliter aliquot of sterilized urine was then added to all tubes containing the above-described antimicrobial factor deactivation system urine cocktail and to an equal number of tubes without the urine cocktail. Culture media containing *Enterobacter cloacae,* ATCC #1344-2, was adjusted to a McFarlin of 0.5, representing approximately $5 \times 10^8$ microorganisms per milliliter of culture media, and then diluted 1:100 in sterile culture media. A 0.1 milliliter aliquot of diluted microorganisms was added to all urine containing tubes and the resulting mixture vigorously agitated. Ten microliter aliquots of the mixture were plated on agar plates such as described in Example 1 above. The remaining mixture was allowed to stand at room temperature for 24 hours after which time, a second ten microliter aliquot was plated as described in Example 1 above. All plating was done in duplicate and the survival index (S-Factor) was calculated for each as described in the Examples above. The average S-Factor for each time point was determined and is recorded in Table 39 below.

TABLE 39
Enterobacter cloacae
(ATCC #1344-2) S-Factor

| Sample | ADS* | NaCl (%)** | 0 Hour | 24 Hours |
|---|---|---|---|---|
| 1 | — | —1.00 | TNTC*** | |
| 2 | + | —1.00 | 1.72 | |
| 3 | + | 1 1.00 | 1.27 | |
| 4 | + | 2 1.00 | .80 | |
| 5 | + | 4 1.00 | 1.03 | |
| 6 | + | 8 1.00 | 0.69 | |

*ADS Antimicrobial factor deactivation system urine cocktail.
**percentage, percentage sodium chloride by weight.
***TNTC too numerous to count.

In the absence of the antimicrobial factor deactivation system urine cocktail, see Sample 1 in Table 39 above, the microorganisms present in the urine will quickly multiply and thus prevent the clinician from obtaining an accurate count of the number of microorganisms per milliliter of urine.

The results, displayed in Table 39 above, indicate that while the antimicrobial factor deactivation system urine cocktail can decrease the rate of microbial replication, the addition of such salts as sodium chloride increase the effectiveness of the urine cocktail in holding the bacterial count, in urine, stable over a 24-hour period. The preferred range of salt, as determined from the results displayed in Table 39 above, is from about 2.5 percent to about 4.0 percent, by weight thereof.

From the results of the salt titration experiment above, it was concluded that addition of about three (3) percent, by weight, sodium chloride to the antimicrobial factor deactivation system urine cocktail should prevent the overgrowth of *Enterobacter cloacae* in urine over a 24-hour period. To verify this conclusion, the antimicrobial factor deactivation system urine cocktail prepared as described above, was added to a series of tubes. A second series of tubes was prepared by adding the identical cocktail plus 0.15 grams of sodium chloride, the equivalent of three (3) percent, by weight, sodium chloride. A third series of tubes were set aside without cocktail. A five-milliliter aliquot of sterile urine was then added to each tube, including those tubes without cocktail. Four strains of *Enterobacter cloacae*, identified in Table 40 below, were grown separately and adjusted to a McFarlin of 0.5. Each strain was then diluted 1:100 in sterile culture media and a 0.1 milliliter aliquot of each added to individual urine tubes as identified in Table 40 below. After vigorous mixing, a ten microliter aliquot from each tube was plated on agar plates as described in Example 8. Thereafter, the mixture was allowed to stand at room temperature and at the time intervals indicated in Table 40 below, another ten (10) microliter aliquot was plated as described in Example 1. The results of each time point given in Table 40 below represents an average survival index (S-Factor) for quadruplicate plating.

The results, given in Table 40 below, confirm an increased stabilization of colony formation for all four strains of *Enterobacter cloacae* afforded by addition of about three percent, by weight, sodium chloride to the antimicrobial deactivation system cocktail.

TABLE 40

*Enterobacter cloacae*
(ATCC: various strains)

S-Factor
hour time points*

| Strain | − | 4 +ADS | +ADS+ NaCl | − | 6 +ADS | +ADS+ NaCl | − | 24 +ADS | +ADS+ NaCl | − | 48 +ADS | +ADS+ NaCl |
|---|---|---|---|---|---|---|---|---|---|---|---|---|
| 1344-2 | 3.2 | 1.28 | 1.97 | 4.75-TN | 1.23 | .91 | TN | 3.88-TN | 2.05 | TN | TN | 4.64-TN |
| 3118 | 3.00 | .87 | .98 | 3.00-TN | .95 | 1.05 | TN | 4.25-TN | .98 | TN | TN | 4.14 |
| 2294 | 2.5-TN** | 1.00 | .76 | 5.53-TN | .89 | .89 | TN | 2.38-TN | .82 | TN | TN | 2.25 |
| 0879 | 2.5 | .95 | 1.03 | 4.02-TN | .86 | 1.06 | TN | TN | 1.07 | TN | TN | 1.29-TN |

*"−" indicates urine without antimicrobial deactivation system cocktail; "+ADS" indicates urine plus antimicrobial deactivation cocktail; "+ADS+NaCl" indicates antimicrobial deactivation system cocktail containing 3 percent, by weight thereof, sodium chloride.
**TN=too numerous to count.

Example 10

This example was performed to test the ability of the antimicrobial factor deactivation system urine cocktail containing three percent, by weight thereof, sodium chloride to hold the colony count of the different microorganisms, listed in Tables 41—43 below, stable in the presence and absence of the antibiotics, listed in Tables 41—43 below, over 24 hours.

The following dry mixture was placed in a series of sterile tubes:

0.03 grams sodium polyanetholsulfonate,

0.005 grams thioglycolate,

0.1 gram of ICN free-base cysteine,

0.1 gram of sodium bicarbonate,

0.15 grams of sodium chloride.

The various antibiotics, listed in Tables 41—43 below, were added, at the concentrations also specified in Tables 41—43 below, to the tubes containing the above-described antimicrobial factor deactivation urine cocktail and to an equal number of tubes without the urine cocktail. Five milliliters of sterile urine was then added to all tubes after which the tubes were vigorously mixed. Control tubes contained either urine alone or urine plus the above-described antimicrobial factor deactivation urine cocktail. No antibiotics were added to control tubes. The microorganisms listed in Tables 41—43 below were adjusted to a McFarlin of 0.5 and then diluted 1:100 with sterile culture media. A 0.1 milliliter aliquot of a single microorganism was added to each urine containing tube and the resultant mixture vigorously agitated. A ten microliter aliquot from each tube containing the resultant mixture was immediately plated as described in Example 8 above. The tubes were then allowed to stand at room temperature for 24 hours. Additional ten microliter aliquots were plated as described in Example 8 above at the time points indicated in Tables 41—43 below. All plating was done in quadruplicate and the S-Factor recorded in Tables 41—43 below represent an average of the quadruplicate plating.

The results set forth in Tables 41—43 indicate that the salt containing antimicrobial deactivation system urine cocktail was able to hold the colony count of *Proteus vulgaria, Streptococcus pneumoniae,* and *Streptococcus pyogenes* relatively stable in both the presence and absence of most antibiotics.

TABLE 41

I. *Proteus vulgaris* (ATCC #23315)

| | S-factor hour time points* | | | | | |
|---|---|---|---|---|---|---|
| | 4 | | 6 | | 24 | |
| Antibiotic** | + | − | + | − | + | − |
| No drug | 1.06 | 1.07 | 1.38 | 3.15 | 1.12 | TNTC |
| Ampicillin (210 µg) | .77 | .19 | 1.19 | .04 | .87 | .004 |
| Cefoxitin (250 µg) | .73 | .002 | .79 | .0008 | .25 | 0 |
| Chloramphenicol (180 µg) | 1.01 | .53 | .56 | .32 | .46 | .06 |
| Erythromycin (80 µg) | .79 | 1.13 | 1.50 | 1.78 | .94 | 1.07-TNTC |
| Gantrisin (1000 µg) | 1.47 | .56 | 1.28 | 1.32 | 1.19 | TNTC |
| Mezlocillin (500 µg) | 2.14 | .31 | 1.51 | .50 | 1.31 | 0 |

—Time point not included in reconstruction.

*Antimicrobial factor deactivation system urine cocktail is present.

**Number in parenthesis represents final concentration of antibiotic per milliliter of urine.

TNTC=too numerous to count.

TABLE 42
II. *Streptococcus pneumoniae* (ATCC #6301)

| Antibiotic** | S-Factor hour time points* | | | | | |
|---|---|---|---|---|---|---|
| | 4 | | 6 | | 24 | |
| | + | − | + | − | + | − |
| No drug | 1.19 | .82 | .95 | .73 | 1.27 | 4.80 |
| Ampicillin (210 µg) | 1.60 | .54 | 1.34 | .43 | 1.08 | .07 |
| Cefamandole (200 µg) | .42 | 1.04 | .75 | 1.12 | 1.36 | .28 |
| Cefoxitin (250 µg) | 1.08 | .98 | 1.11 | .94 | 1.01 | .20 |
| Cephalothin (200 µg) | 1.04 | .53 | 1.42 | .53 | 2.54 | .21 |
| Chloramphenicol (180 µg) | .57 | .77 | .80 | .31 | 1.03 | .30 |
| Erythromycin (80 µg) | .99 | .73 | .83 | .73 | .78 | .32 |
| Gantrisin (1000 µg) | 1.00 | .96 | 1.00 | .96 | 1.10 | 14.54 |
| Mezlocillin (500 µg) | 1.18 | .71 | 1.05 | .29 | 1.03 | .01 |

−Time point not included in reconstruction.
*Antimicrobial factor deactivation system urine cocktail is present.
**Number in parenthesis represents final concentration of antibiotic per milliliter of urine.
TNTC=too numerous to count.

TABLE 43
III. *Streptococcus pyogenes* (ATCC #19615)

| Antibiotic** | S-Factor hour time points* | | | | | |
|---|---|---|---|---|---|---|
| | 4 | | 6 | | 24 | |
| | + | − | + | − | + | − |
| No drug | 1.02 | 1.95 | .93 | 3.10 | .84 | TNTC** |
| Ampicillin (210 µg) | .90 | 1.50 | 1.00 | 1.50 | .79 | 1.00 |
| Cefamandole (200 µg) | .52 | .41 | .88 | .09 | 1.28 | .26 |
| Cefoxitin (250 µg) | .60 | .30 | .94 | .24 | .96 | .10 |
| Cephalothin (200 µg) | 1.35 | .63 | 1.50 | .58 | 1.62 | .08 |
| Chloramphenicol (180 µg) | .84 | 1.15 | 1.05 | 1.06 | .75 | .22 |
| Erythromycin (80 µg) | .54 | .97 | .33 | .81 | .54 | .76 |
| Gantrisin (1000 µg) | .84 | 2.01 | .94 | 2.34 | .78 | 1.56-TNTC |
| Mezlocillin (500 µg) | .95 | .74 | 1.33 | .51 | .62 | .10 |

−Time point not included in reconstruction.
*Antimicrobial factor deactivation system urine cocktail is present.
**Number in parenthesis represents final concentration of antibiotic per milliliter of urine.
TNTC=too numerous to count.

Example 11

This example was performed to establish the effective concentration range of sodium polyanetholsulfonate (SPS) in the antimicrobial factor deactivation system urine cocktail in the presence of the various antibiotics listed in Table 44 below.

The following dry mixture was placed in each of a series of sterile tubes:

0.005 grams thioglycolate,
0.1 gram ICN free-base cysteine,
01. gram sodium bicaronate,
0.15 grams sodium chloride.

Different amounts of SPS, designated by weight percent thereof in Table 44 below, were added to the tubes containing the above-described antimicrobial factor deactivation system urine cocktail and to the control tubes. The various antibiotics listed in Table 44 below were then added to one half of the tubes containing the above-described antimicrobial factor deactivation urine cocktail. Five milliliter aliquots of sterile urine were next added to all tubes. Control tubes containing urine but no antibiotic were established, half of which contained the above-described antimicrobial factor deactivation system urine cocktail plus the various amounts of SPS designated in Table 44 below. *Staphylococcus aureus*, ATCC #25923, were grown, prepared and aliquoted into all tubes as described in Example 8 above. All tubes were plated as described in Example 8 above at the time points indicated in Table 44 below.

TABLE 44
*Staphylococcus aureus* (ATCC #25923)

| | | S-Factor hour time points* | | | | | | | |
|---|---|---|---|---|---|---|---|---|---|
| | | 2 | | 4 | | 6 | | 24 | |
| SPS%*** | Antibiotic** | + | − | + | − | + | − | + | − |
| — | No drug | ND | ND | ND | 1.39 | ND | 1.50 | ND | 2.3-TNTC**** |
| 0.6 | No drug | .70 | ND | 6.0 | ND | ND | ND | .44 | ND |
| 1.0 | No drug | ND | ND | 1.4 | ND | .83 | ND | .66 | ND |
| 2.0 | No drug | ND | ND | .85 | ND | .83 | ND | .91 | ND |
| 6.0 | No drug | .87 | ND | .76 | ND | ND | ND | .63 | ND |
| 0.6 | Gentamicin (60 µg) | .63 | .02 | .51 | 0 | ND | ND | .51 | 0 |
| 1.0 | Gentamicin (60 µg) | ND | ND | 1.12 | 0 | 1.03 | .04 | 1.22 | 0 |
| 2.0 | Gentamicin (60 µg) | ND | ND | .75 | 0 | .75 | 0 | .47 | 0 |
| 6.0 | Gentamicin (60 µg) | .87 | .12 | .76 | .01 | ND | ND | .53 | 0 |
| 0.6 | Tetracycline (90 µg) | .64 | .07 | .73 | .01 | ND | ND | .6 | 0 |
| 1.0 | Tetracycline (90 µg) | ND | ND | 1.0 | 0.1 | 1.3 | 0 | .67 | 0 |
| 2.0 | Tetracycline (90 µg) | ND | ND | .68 | 0 | .74 | .007 | .5 | .002 |
| 6.0 | Tetracycline (90 µg) | .91 | 1.09 | .87 | .34 | ND | ND | ND | ND |

—Time point not included in reconstruction.
*Antimicrobial factor deactivation system urine cocktail is present.
**Number in parenthesis represents final concentration of antibiotic per milliliter of urine.
***Final sodium polyetholsulfonate concentration, by weight thereof, per tube.
****TNTC=too numerous to count.
+Contains Antimicrobial Factor Deactivation System.
−Does not contain Antimicrobial Factor Deactivation System.

The results set forth in Table 44 above reveal an optimum range for SPS in the antimicrobial deactivation system urine cocktail to be between about 0.6% and about 2.0%, by weight thereof.

# EP 0 107 070 B1

**Claims**

1. A method for preserving the microbial integrity of a specimen from a time of specimen collection to a time of specimen analysis, comprising the steps of
   (a) collecting said specimen and
   (b) admixing with said specimen to form a treated specimen, a system deactivating antimicrobial factors present in said specimen so that at least some microorganisms of interest will be capable of replicating upon a dilution of said treated specimen on media capable of supporting replication of said microorganisms of interest, said system comprising sodium polyanetholsulfonate or sodium amylosulfate in an amount of 0.3 to 1% by weight of said system and said specimen combined.

2. A method for preserving the microbial integrity of a specimen from a time of specimen collection to a time of specimen analysis, comrpising the steps of
   (a) collecting said specimen and
   (b) admixing with said specimen to form a treated specimen, a system deactivating antimicrobial factors so that at least some microorganisms of interest will be capable of replicating upon dilution of said treated specimen on media capable of supporting replication of said microorganisms of interest, said system comprising a combination of sodium polyanetholsulfonate or sodium amylosulfate with a substance selected from the group consisting of a composition inhibiting the action of penicillin, streptomycin and cephalosporins, a sulfur compound deactivating agent and antibiotic specific enzymes or mixtures of the same, whereby the amount of polyanetholsulfonate or sodium amylosulfate in the combination is 0.1 to 1% by weight of said system and said specimen combined.

3. The method of claims 1 or 2, wherein step (b) comprises admixing with said specimen a system deactivating antimicrobial factors and stabilizing the number of microorganisms of interest present in said specimen.

4. The method of claims 1 to 3, wherein said specimen is selected from blood, bone marrow, spinal fluid, sputum, pleural fluid, body secretions and urine.

5. The method of claims 2 to 4, wherein the composition inhibiting the action of penicillin, streptomycin and cephalosporins is a substance selected from the group consisting of thioglycolate, glutathion and N-acetyl-cysteine.

6. The method of claims 1 to 5, wherein the system contains cysteine or a mixture of thioglycolate and cysteine.

7. The method of claims 2 to 6, wherein the sulfa compound deactivating agent is para-aminobenzoic acid in an amount of from about 5 micrograms per milliliter to about 500 micrograms per milliliter of said treating fluid and said sample fluid.

8. In a method of detecting microbial pathogens within a sample fluid suspected of containing microbial pathogens and at least one antimicrobial factor wherein said sample fluid is placed within a confined sterile centrifugation zone and subjected to centrifugation to thereby concentrate said microbial pathogens and thereafter separating said concentrated microbial pathogens from said centrifugation zone and analyzing same for said microbial pathogens, the improvement comprising:
   preventing said antimicrobial factor from attacking said microbial pathogen while said sample is in said confined sterile centrifugation zone and prior to said analysis by incorporating an effective deactivating amount of an antimicrobial deactivation agent which is noncidal to said microbial pathogens within said confined sterile centrifugation zone such that it is thoroughly admixed with said sample when said sample fluid is placed within said confined sterile centrifugation zone, said antimicrobial deactivation agent comprising sodium polyanetholsulfonate or sodium amylosulfate in an amount in the range of from 0.1 to 1.0% by weight of said specimen fluid and said treating fluid, cysteine in an amount of 0.05 to 2.5% by weight of said specimen fluid and said treating fluid and thioglycolate in an amount of from 0.05 to 1.6% by weight of said specimen fluid and said treating fluid.

9. The method of claim 8, wherein the antimicrobial factor deactivating agent further comprises para-aminobenzoic acid in an amount of from about 5 micrograms per milliliter to about 500 micrograms per milliliter of said treating fluid and said sample fluid.

10. A method of detecting microbial pathogens in a blood sample which is suspected to contain said microbial pathogens and antimicrobial factors including at least one antibiotic drug comprising:
   (a) depositing said blood sample in a confined sterile zone in contact with an aqueous blood treating media which contains a lysing agent and an antimicrobial factor deactivating system including an effective quantity of a deactivating agent for said antibiotic drug which is noncidal to said microbial pathogens, said antimicrobial factor deactivating system comprising from 0.1 to 6% by weight thereof of sodium polyanetholsulfonate or sodium amylosulfate, from 0.05 to 2.5% by weight of cysteine, from 0.05 to 1.6% by weight of thioglycolate;
   (b) admixing said blood sample with said blood treating media to allow said blood treating medium to treat said blood sample;
   (c) subjecting said confined sterile zone to centrifugation thereby concentrating said microbial pathogens; and
   (d) separating said concentrated microbial pathogens from said confined sterile zone and subjecting them to identification analysis.

46

## EP 0 107 070 B1

11. The method of claim 10, wherein said identification comprises diluting said concentrated microbial pathogens on growth media therefor in a ratio of at least about 1:60 and thereafter allowing said microbial pathogens to culture.

12. The method of claims 10 or 11, wherein sodium polyanetholsulfonate or sodium amylosulfate is present in a quantity of from 0.1 to 1.0% by weight of said sample fluid and said treating fluid.

13. The method of claims 10 or 11, wherein sodium polyanetholsulfonate or sodium amylosulfate is present in a quantity of from about 0.3 to about 0.6% by weight of said sample fluid and said treating fluid.

14. The method of claims 10 to 13, wherein thioglycolate is present in an amount of from 0.5 to 6% of said sample fluid and said treating fluid.

15. A method of detecting microbial pathogens within a urine sample suspected of containing microbial pathogens and at least one antimicrobial factor comprising:

(a) depositing said sample fluid or portion thereof in a confined sterile zone in contact with an antimicrobial factor deactivating system including sodium polyanetholsulfonate or sodium amylosulfate in an amount of 0.6 to 6% by weight of sample fluid;

(b) admixing said urine sample with said antimicrobial factor deactivating system; and

(c) subjecting said admixture to identification analysis.

16. The method of claim 15, wherein said antimicrobial factor deactivating system includes penicillin and cephalosporin deactivating agent.

17. The method of claims 15 or 16 wherein said penicillin and cephalosporin deactivating agent is cysteine and thioglycolate.

18. The method of claims 15 to 17 wherein said antimicrobial factor deactivating system includes sodium polyanetholsulfonate in an amount in the range of from about 0.6% to about 2.0% by weight of said sample fluid; cysteine in an amount of from about 0.5% to about 2.5% by weight of said sample fluid; thioglycolate in an amount of about 0.1% by weight of said sample fluid; sodium bicaronate in an amount of about 2.0% by weight of sample fluid; and said sample fluid.

19. The method of claim 18 wherein a salt is included in said antimicrobial factor deactivating system.

20. The method of claim 19 wherein said salt is sodium chloride.

21. The method of claim 20 wherein said sodium chloride is present in an amount in the range of from about 2.5% to about 4.0% by weight of said sample fluid.

22. An article for use in the methods of claims 1 to 21 for the concentration of microbial pathogens from a sample fluid comprising:

an enclosed centrifugation receptacle suitably closed with injectable closure means, the interior of said receptacle comprising an evacuated space maintained at a lower than atmospheric pressure which will draw a predetermined weight of sample fluid therein and adjacent a high density nontoxic to microbial pathogens water immiscible hydrophobic liquid cushioning agent capable of collecting substantially all of the microbial pathogens which pass out of suspension from said sample fluid without a loss of said microbial pathogens to interstitial spaces present in said centrifugation receptacle and where the density of the liquid cushioning agent is sufficient so as not to be supported by a mixture of sample fluid and a treating fluid therefor, the treating fluid comprising a lysing agent and the antimicrobial factor deactivating agent according to claims 1 to 21.

23. Article according to claim 22 wherein the liquid cushioning agent is a fluorinated hydrocarbon having a boiling point in the range of from 93°C to 216°C.

24. Article according to claim 22 or 23 wherein the antimicrobial deactivator system is in the form of solid particles.

25. An article for use in the methods of claims 1 to 21 for the collection and treatment of microbial pathogens from the sample fluid comprising

a receptacle containing a sample treating material which comprises an antimicrobial factor deactivation system according to claims 1 to 21.

26. The article of claim 25 wherein said article comprises an elongated flexible tube with a first enclosed end and a second open end;

a removal closure member for said second end;

a crushable ampule positioned therein adjacent said closed end and containing a liquid growth media therein; a body of absorbent material positioned adjacent said ampule and between said ampule and said general open end and containing said antimicrobial factor deactivation system as solid particles according to claims 1 to 21 distributed therewithin;

a swab comprising sorbent material contained on one end thereof and positioned adjacent said body of sorbent material and connected to a handle which extends therefrom to said second open end.

27. The article of claim 26 wherein said handle of said swab which extends to said second open end is connected to said removable closure member.

**Patentansprüche**

1. Verfahren zum Schützen der mikrobiologischen Integrität einer Probe vom Zeitpunkt der Probenentnahme bis zum Zeitpunkt der Probenanalyse, dadurch gekennzeichnet, daß man

(a) die Probe entnimmt und

EP 0 107 070 B1

(b) die Probe zur Bildung einer behandelten Probe mit einem System vermischt, welches in der Probe enthaltene antimikrobielle Faktoren inaktiviert, so daß mindestens einige der interessierenden Mikroorganismen nach Verdünnen der behandelten Probe zur Replikation auf Medien befähigt sind, welche geeignet sind, die Replikation der interessierenden Mikroorganismen zu fördern, wobei das System Natriumpolyanetholsulfonat oder Natriumamylosulfat in einer Menge von 0,3 bis 1 Gew.%, bezogen auf die Kombination aus dem System und der Probe, enthält.

2. Verfahren zum Schützen der mikrobiologischen Integrität einer Probe vom Zeitpunkt der Probenentnahme bis zum Zeitpunkt der Probenanalyse, dadurch gekennzeichnet, daß man

(a) die Probe entnimmt und

(b) die Probe zur Bildung einer behandelten Probe mit einem System vermischt, welches in der Probe enthaltene Antimikrobielle Faktoren inaktiviert, so daß mindestens einige der interessierenden Mikroorganismen nach Verdünnen der behandelten Probe zur Replikation auf Medien befähigt sind, welche geeignet sind, die Replikation der interessierenden Mikroorganismen zu fördern, wobei das System eine Kombination von Natriumpolyanetholsulfonat oder Natriumamylosulfat mit einer Substanz enthält, welche ausgewählt ist aus der Gruppe bestehend aus einer die Wirkung von Penicillin, Streptomycin und Cephalosporinen inhibierenden Substanz, einem Schwefelverbindungen inaktivierenden Mittel und antibiotikaspezifischen Enzymen oder Mischungen derselben, wobei die Menge von Polyanetholsulfonat oder Natriumamylosulfat in der Kombination 0,1 bis 1 Gew.%, bezogen auf die Kombination aus dem System und der Probe, beträgt.

3. Verfahren nach den Ansprüchen 1 oder 2, dadurch gekennzeichnet, daß man in Stufe (b) ein System mit der Probe vermischt, welches antimikrobielle Faktoren inaktiviert und die Zahl der interessierenden Mikroorganismen, welche in der Probe vorhanden sind, stabilisiert.

4. Verfahren nach den Ansprüchen 1 bis 3, dadurch gekennzeichnet, daß man eine Probe verwendet, welche ausgewählt ist aus Blut, Knochenmark, Rückenmarksflüssigkeit, Sputum, Pleura-Flüssigkeit, Körperausscheidungen und Urin.

5. Verfahren nach den Ansprüchen 2 bis 4, dadurch gekennzeichnet, daß die die Wirkung von Penicillin, Streptomycin und Cephalosporinen inhibierende Verbindung eine Substanz ausgewählt aus der Gruppe bestehend aus Thioglykolat, Glutation und N-Acetyl-Cystein ist.

6. Verfahren nach den Ansprüche 1 bis 5, dadurch gekennzeichnet, daß das System Cystein oder eine Mischung aus Thioglykolat und Cystein enthält.

7. Verfahren nach den Ansprüchen 2 bis 6, dadurch gekennzeichnet, daß das Schwefelverbindungen inaktivierende Mittel para-Aminobenzoesäure in einer Menge von etwa 5 bis 100 µg/ml der Behandlungsflüssigkeit und der Probenflüssigkeit ist.

8. Verfahren zum Nachweis von mikrobiellen Pathogenen in einer Probenflüssigkeit, von der man annimmt, daß sie mikrobielle Pathogene und mindestens einen antimikrobiellen Faktor enthält, wobei man die Flüssigkeitsprobe in eine geschlossene Zentrifugationszone einbringt und zentrifugiert, um auf diese Weise mikrobielle Pathogene zu konzentrieren und anschließend der konzentrierten mikrobiellen Pathogene aus der Zentrifugationszone abtrennt und die mikrobiellen Pathogene analysiert, dadurch gekennzeichnet, daß man verhindert, daß der antimikrobielle Faktor die mikrobiellen Pathogene angreift, während die Probe sich in der geschlossenen sterilen Zentrifugationszone und in dem Stadium vor der Analyse befindet, indem man eine zum Inaktivieren ausreichende Menge eines antimikrobiellen Inaktivierungsmittels, welches die mikrobiellen Pathogene nicht abtötet, in die geschlossene sterile Zentrifugationszone einbringt, so daß dieses vollständig mit der Probe vermischt wird, wenn die Probenflüssigkeit in die abgeschlossene sterile Zentrifugationszone eingeführt wird, wobei das antimikrobielle Inaktivierungsmittel Natriumpolyanetholsulfonat oder Natriumamylosulfat in einer Menge im Bereich von 0,1 bis 1 Gew.%, bezogen auf die Probenflüssigkeit und die Behandlungsflüssigkeit, Cystein in einer Menge von 0,05 bis 2,5 Gew.%, bezogen auf Probenflüssigkeit und Behandlungsflüssigkeit, und Thioglykolat in einer Menge von 0,05 bis 1,6 Gew.%, bezogen auf Probenflüssigkeit und Behandlungsflüssigkeit, enthält.

9. Verfahren nach Anspruch 8, dadurch gekennzeichnet, daß das antimikrobielle Faktoren inaktivierende Mittel ferner para-Aminobenzoesäure in einer Menge von etwa 5 bis 500 µg/ml der Behandlungsflüssigkeit und der Probenflüssigkeit enthält.

10. Verfahren zum Nachweis von mikrobiellen Pathogenen in einer Blutprobe, von welcher man annimmt, daß sie mikrobielle Pathogene und antimikrobielle Faktoren einschließlich mindestens eines Antibiotikums enthält, dadurch gekennzeichnet, daß man

(a) die Blutprobe in einer geschlossenen sterilen Zone in Kontakt mit einem wässrigen Blutbehandlungsmedium bringt, welches ein lysierendes Mittel und ein antimikrobielle Faktoren aktivierendes System einschließlich einer wirksamen Menge eines Inaktivierungsmittels für das Antibiotikum enthält, welches die mikrobiellen Pathogene nicht abtötet, wobei das antimikrobielle Faktoren inaktivierende System 0,1 bis 6 Gew.% Natriumpolyanetholsulfonat oder Natriumamylosulfat, 0,05 bis 2,5 Gew.% Cystein und 0,05 bis 1,6 Gew.% Thioglykolat enthält,

(b) die Blutprobe mit dem Blutbehandlungsmittel vermischt, um das Blutbehandlungsmittel auf die Blutprobe einwirken zu lassen,

(c) die geschlossene sterile Zone zentrifugiert, um auf diese Weise die mikrobiellen Pathogene zu konzentrieren und

48

# EP 0 107 070 B1

(d) die konzentrierten mikrobiellen Pathogene aus der geschlossenen sterilen Zone abtrennt und sie einer Identifikationsanalyse unterwirft.

11. Verfahren nach Anspruch 10, dadurch gekennzeichnet, daß man die konzentrierten mikrobiellen Pathogene für die Identifikation auf einem geeigneten Wachstumsmedium in einem Verhältnis von mindestens etwa 1:60 verdünnt und anschließend die mikrobiellen Pathogene kultiviert.

12. Verfahren nach den Ansprüchen 10 oder 11, dadurch gekennzeichnet, daß Natriumpolyanetholsulfonat oder Natriumamylosulfat in einer Menge von 0,1 bis 1,0 Gew.%, bezogen auf die Probenflüssigkeit und die Behandlungsflüssigkeit, vorhanden sind.

13. Verfahren nach den Ansprüchen 10 oder 11, dadurch gekennzeichnet, daß Polyanetholsulfonat oder Natriumamylosulfat in einer Menge von etwa 0,3 bis 0,6 Gew.%, bezogen auf die Probenflüssigkeit und die Behandlungsflüssigkeit, vorhanden sind.

14. Verfahren nach den Ansprüchen 10 bis 13, dadurch gekennzeichnet, daß Thioglykolat in einer Menge von 0,5 bis 6 Gew.%, bezogen auf die Probenflüssigkeit und die Behandlungsflüssigkeit, vorhanden ist.

15. Verfahren zum Nachweise von mikrobiellen Pathogenen in einer Urinprobe, von der man annimmt, daß sie mikrobielle Pathogene und mindestens einen antimikrobiellen Faktor enthält, dadurch gekennzeichnet, daß man

(a) die Flüssigkeitsprobe oder einen Teil derselben in einer geschlossenen sterilen Zone in Kontakt mit einem antimikrobielle Faktoren inaktivierenden System bringt, welches Natriumpolyanetholsulfonat oder Natriumamylosulfat in einer Menge von 0,6 bis 6 Gew.%, bezogen auf die Probenflüssigkeit, enthält,

(b) die Urinprobe mit dem antimikrobielle Faktoren inaktivierenden System vermischt und

(c) die Mischung einer Identifikationsanalyse unterwirft.

16. Verfahren nach Anspruch 15, dadurch gekennzeichnet, daß das antimikrobielle Faktoren inaktivierende System Penicillin und Cephalosporin inaktivierende Mittel einschließt.

17. Verfahren nach den Ansprüchen 15 oder 16, dadurch gekennzeichnet, daß das Penicillin und Cephalosporin inaktivierende Mittel Cystein oder Thioglykolat ist.

18. Verfahren nach den Ansprüchen 15 bis 17, dadurch gekennzeichnet, daß das antimikrobielle Faktoren inaktivierende System Natriumpolyanetholsulfonat in einer Menge von etwa 0,6 bis etwa 2,0 Gew.%, bezogen auf die Flüssigkeitsprobe, Cystein in einer Menge von etwa 0,5 bis etwa 2,5 Gew.%, bezogen auf die Flüssigkeitsprobe, Thioglykolat in einer Menge von etwa 0,1 Gew.%, bezogen auf die Flüssigkeitsprobe, Natriumbicarbonat in einer Menge von etwa 2,0 Gew.%, bezogen auf die Flüssigkeitsprobe und die Flüssigkeitsprobe enthält.

19. Verfharen nach Anspruch 18, dadurch gekennzeichnet, daß das antimikrobielle Faktoren inaktivierende System ein Salz enthält.

20. Verfahren nach Anspruch 19, dadurch gekennzeichnet, daß das Salz Natriumchlorid ist.

21. Verfahren nach Anspruch 20, dadurch gekennzeichnet, daß Natriumchlorid in einer Menge von etwa 2,5 bis etwa 4,0 Gew.%, bezogen auf die Probenflüssigkeit, vorhanden ist.

22. Vorrichtung zur Verwendung in den Verfahren nach den Ansprüchen 1 bis 21 zur Konzentration der mikrobiellen Pathogene aus einer Probenflüssigkeit, gekennzeichnet durch ein geschlossenes Zentrifugationsgefäß, welches mit geeigneten durchstechbaren Verschlußteilen verschlossen ist, wobei das Innere des Gefäßes einen evakuierten Raum enthält, der bei niedrigerem als atmosphärischem Druck gehalten wird und in der Lage ist, ein vorbestimmtes Gewicht der Probenflüssigkeit in das Innere und angrenzend an ein gegenüber mikrobiellen Pathogenen nicht toxisches, mit Wasser nicht mischbares, hydrophobes, flüssiges Kissen bildendes Mittel mit hoher Dichte zu saugen, welches im wesentlichen alle mikrobiellen Keime, die aus der Suspension der Flüssigkeitsprobe austreten, aufnehmen kann, ohne daß mikrobielle Keime in den Zwischenräumen verlorengehen, welche in dem Zentrifugengefäß vorhanden sind, und die Dichte des flüssigen Kissen bildenden Mittels so groß ist, daß es nicht von einer Mischung aus Probenflüssigkeit und einer dafür vorgesehenen Behandlungsflüssigkeit unterschichtet werden kann, wobei die Behandlungsflüssigkeit ein lysierendes Mittel und das antimikrobielle Faktoren inaktivierende Mittel nach den Ansprüchen 1 bis 21 enthält.

23. Vorrichtung nach Anspruch 22, dadurch gekennzeichnet, daß das flüssige Kissen bildende Mittel ein fluorierter Kohlenwasserstoff mit einem Siedepunkt im Bereich von 93 bis 216°C ist.

24. Vorrichtung nach den Ansprüchen 22 oder 23, dadurch gekennzeichnet, daß das antimikrobielle Inaktivierungssystem in Form von festen Partikeln vorliegt.

25. Vorrichtung zur Verwendung in den Verfahren nach den Ansprüchen 1 bis 21 zum Sammeln und zum Behandeln von mikrobiellen Pathogenen aus Probenflüssigkeiten, gekennzeichnet durch ein Gefäß mit einem Gehalt an einem Material zur Behandlung von Proben, welches ein antimikrobielle Faktoren inaktivierendes System nach den Ansprüchen 1 bis 21 enthält.

26. Vorrichtung nach Anspruch 25, gekennzeichnet durch eine längliche, flexible Röhre mit einem ersten geschlossenen Ende und einem zweiten offenen Ende,

einem abnehmbaren Verschlußteil für das offene Ende, einer angrenzend an das geschlossene Ende angeordneten zerbrechbaren Ampulle, welche ein flüssiges Wachstumsmedium enthält,

einen angrenzend an die Ampulle und zwischen Ampulle und dem offenen Ende angeordneten Körper aus einem absorbierenden Material, in welchem das antimikrobielle Faktoren inaktivierende System nach den Ansprüchen 1 bis 21 in Form von festen Partikeln verteilt vorliegt,

49

## EP 0 107 070 B1

einen Tupfer, welcher an seinem einen Ende absorbierendes Material aufweist und angrenzend an den Körper aus absorbierendem Material angeordnet und mit einem Griff verbunden ist, welcher sich bis zu dem zweiten offenen Ende erstreckt.

27. Vorrichtung nach Anspruch 26, dadurch gekennzeichnet, daß der Griff des Tupfers, welcher sich bis zu dem zweiten offenen Ende erstreckt, mit dem abnehmbaren Verschlußteil verbunden ist.

**Revendications**

1. Procédé pour conserver l'intégrité microbienne d'un échantillon depuis le moment du prélèvement de cet échantillon jusqu'au moment de l'analyse de cet échantillon, comprenant les opérations de:
   a) prélevèment dudit échantillon et,
   b) mélange dudit échantillon pour former un échantillon traité, un système désactivant les facteurs antimicrobiens présents dans ledit échantillon de façon qu'au moins certains micro-organismes intéressants soient capables de se répliquer après dilution dudit échantillon traité sur des milieux capables de supporter la réplication desdits micro-organismes intéressants, ledit système comprenant du polyanéthol sulfonate de sodium ou de l'amylosulfate de sodium en une quantité représentant de 0,3 à 1% en poids dudit système et dudit échantillon combinés.

2. Procédé pour conserver l'intégrité microbienne d'un échantillon depuis le moment du prélèvement de cet échantillon jusqu'au moment de l'analyse de cet échantillon, comprenant les opérations de:
   a) prélèvement de cet échantillon et,
   b) mélange dudit échantillon pour former un échantillon traité, un système désactivant les facteurs antimicrobiens de façon qu'au moins certains micro-organismes intéressants soient capables de se répliquer lors d'une dilution dudit échantillon traité sur des milieux capables de supporter la réplication desdits micro-organismes intéressants, ledit système comprenant une combinaison de polyanéthol sulfonate de sodium ou d'amylosulfate de sodium avec une substance choisie parmi une composition inhibant l'action de la pénicilline, de la streptomycine et des cephalosporines, un agent désactivant les composés soufrés et des enzymes spécifiques des antibiotiques ou leurs mélanges, la quantité de polyanéthol sulfonate ou d'amylosulfate de sodium dans la combinaison étant de 0,1 à 1% du poids dudit système et dudit échantillon combinés.

3. Procédé selon la revendication 1 ou 2, dans lequel l'opération b) comprend le mélange audit échantillon d'un système désactivant les facteurs antimicrobiens et stabilisant le nombre de micro-organismes intéressants présents dans ledit échantillon.

4. Procédé selon les revendications 1 à 3, dans lequel ledit échantillon est choisi parmi le sang, la moëlle osseuse, le liquide céphalorachidien, les crachats, le fluide pleural, les sécrétions corporelles et l'urine.

5. Procédé selon les revendications 2 à 4, dans lequel la composition inhibant l'action de la pénicilline, de la streptomycine et des céphalosporines est une substance choisie parmi le thioglycolate, le glutathion et la N-acetyl-cystéine.

6. Procédé selon les revendications 1 à 5, dans lequel le système contient de la cystéine ou un mélange de thioglycolate et de cystéine.

7. Procédé selon les revendications 2 à 6, dans lequel l'agent désactivant les composés sulfa est l'acide para-aminobenzoic en une quantité représentant d'environ 5 microgrammes par millilitre à environ 500 microgrammes par millilitre dudit fluide à trater et dudit échantillon de fluide.

8. Dans un procédé de détection d'agents pathogènes microbiens dans un échantillon de fluide suspecté de contenir des agents pathogènes microbiens et au moins un facteur antimicrobien, dans lequel ledit échantillon de fluide est diposé dans une zone de centrifugation stérile confinée et soumis à une centrifugation pour y concentrer lesdits agents pathogènes microbiens, puis lesdits agents pathogènes microbiens concentrés sont séparés de ladite zone de centrifugation et analysés du point de vue des agents pathogènes microbiens, le perfectionnement comprenant:
   —l'empêchement dudit facteur antimicrobien d'attaquer ledit agent pathogène microbien, alors que ledit échantillon est dans ladite zone de centrifugation stérile confinée et avant ladite analyse, par incorporation d'une quantité désactivante efficace d'un agent de désactivation d'antimicrobiens qui n'est pas microbicide vis-à-vis desdits agents pathogènes microbiens, à l'intérieur de ladite zone de centrifugation stérile confinée, de telle façon qu'il est mélange intimement avec ledit échantillon lorsque ledit échantillon de fluide est disposé dans ladite zone de centrifugation stérile confinée, ledit agent de désactivation d'antimicrobiens comprenant du polyanéthol sulfonate de sodium ou de l'amylosulfate de sodium en une quantité représentant de 0,1 à 1,0% en poids dudit échantillon de fluide et dudit fluide de traitement, de la cystéine en une quantité représentant de 0,05 à 2,5% en poids dudit échantillon de fluide et dudit fluide de traitement et du thioglycolate en une quantité représentant de 0,05 à 1,6% en poids dudit échantillon de fluide et dudit fluide de traitement.

9. Procédé selon la revendication 8, dans lequel l'agent de désactivation du facteur antimicrobien comprend en outre de l'acide para-aminobenzoic en une quantité représentant d'environ 5 microgrammes par millilitre à environ 500 microgrammes par millilitre dudit fluide de traitement et dudit échantillon de fluide.

10. Procédé de détection d'agents pathogènes microbiens dans un échantillon de sang qui est suspecté contenir lesdits agents pathogènes microbiens et des facteurs antimicrobiens, comprenant au moins un médicament antibiotique, comprenant:

a) le dépôt dudit échantillon de sang dans une zone stérile confinée en contact avec un milieu de traitement du sang aqueux qui contient un agent de lyse et un système de désactivation de facteurs antimicrobiens, comprenant une quantité efficace d'un agent désactivant pour ledit médicament antibiotique qui n'est pas microbicide pour lesdits agents pathogènes microbiens, ledit système de désactivation du facteur antimicrobien comprenant de 0,1 à 6% en poids de polyanéthol sulfonate de sodium ou d'amylosulfate de sodium, de 0,05 à 2,5% en poids de cystéine et de 0,05 à 1,6% en poids de thioglycolate;

b) le mélange dudit échantillon de sang avec ledit milieu de traitement du sang pour permettre audit milieu de traitement du sang de traiter ledit échantillon de sang;

c) la soumission de ladite zone stérile confinée à une centrifugation pour concentrer lesdits agents pathogènes microbiens; et

d) la séparation desdits agents pathogènes microbiens concentrés de ladite zone stérile confinée et leur soumission à une anlyse d'identification.

11. Procédé selon la revendication 10, dans lequel ladite identification comprend la dilution desdits agents pathogènes microbiens concentrés sur un milieu de culture dans un rapport d'au moins environ 1:60, puis la culture desdits agents pathogènes microbiens.

12. Procédé selon la revendication 10 ou 11, dans lequel le polyanéthol sulfonate de sodium ou l'amylosulfate de sodium est présent dans une quantité représentant de 0,1 à 1% en poids dudit échantillon de fluide et dudit fluide de traitement.

13. Procédé selon la revendication 10 ou 11, dans lequel le polyanéthol sulfonate de sodium ou l'amylosulfate de sodium est présent dans une quantité représentant d'environ 0,3 à environ 0,6% en poids dudit échantillon de fluide et dudit fluide de traitement.

14. Procédé selon les revendications 10 à 13, dans lequel le thioglycolate est présent dans une quantité représentant de 0,5 à 6% dudit échantillon de fluide et dudit fluide de traitement.

15. Procédé de détection d'agents pathogènes microbiens dans un échantillon d'urine suspectée de contenir des agents pathogènes microbiens et au moins un facteur antimicrobien, comprenant:

a) le dépôt dudit échantillon de fluide ou d'une partie de cet échantillon dans une zone stérile confinée en contact avec un système désactivant du facteur antimicrobien, comprenant du polyanéthol sulfonate de sodium ou de l'amylosulfate de sodium en une quantité représentant de 0,6 à 6% du poids de l'échantillon de fluide;

b) le mélange dudit échantillon d'urine avec ledit système de désactivation du facteur antimicrobien et

c) la soumission dudit mélange à une analyse d'identification.

16. Procédé selon la revendication 15, dans lequel ledit système désactivant du facteur antimicrobien comprend un agent désactivant la pénicilline et la céphalosporine.

17. Procédé selon les revendications 15 et 16, dans lequel ledit agent désactivant la pénicilline et la céphalosporine est la cystéine et le thioglycolate.

18. Procédé selon les revendications 15 à 17, dans lequel ledit système désactivant les facteurs antimicrobiens comprend le polyanéthol sulfonate de sodium en une quantité représentant d'environ 0,6 à 2,0% du poids dudit échantillon fluide; de la cystéine dans une quantité représentant d'environ 0,5 à environ 2,5% du poids dudit échantillon de fluide; du thioglycolate en une quantité représentant environ 0,1% du poids dudit échantillon de fluide; du bicarbonate de sodium en une quantité représentant environ 2,0% du poids dudit échantillon de fluide et le echantillon fluide.

19. Procédé selon la revendication 18, dans lequel un sel est compris dans le système de désactivation du facteur antimicrobien.

20. Procédé selon la revendication 19, dans lequel le sel est du chlorure de sodium.

21. Procédé selon la revendication 20, dans lequel le chlorure de sodium est présent en une quantité représentant d'environ 2,5% à environ 4% du poids dudit échantillon de fluide.

22. Article destiné à être utilisé dans un procédé selon les revendications 1 à 21 pour la concentration d'agents pathogènes microbiens d'un échantillon de fluide, comprenant:

—un réceptacle de centrifugation convenablement fermé avec des moyens de fermeture injectables, l'intérieur de ce réceptacle comprenant un espace sous vide maintenu à une pression inférieure à la pression atmosphérique qui aspirera une quantité prédéterminée de l'échantillon de fluide et, adjacent à ce dernier, un agent amortisseur liquide hydrophobe non mixible à l'eau de haute densité non toxique pour les agents pathogènes, capable de recueillir pratiquement tous les agents pathogènes microbiens qui passent hors de la suspension dudit échantillon de fluide sans perte des agents pathogènes microbiens en des espaces intersticiels présents dans ledit réceptacle de centrifugation et où la densité de l'agent amortisseur liquide est suffisante pour ne pas être supportée par un mélange dudit échantillon de fluide et d'un fluide de traitement, le fluide de traitement comprenant un agent de lyse et l'agent désactivant le facteur antimicrobien, selon les revendications 1 à 21.

23. Article selon la revendication 22, dans lequel l'ayant amortisseur liquide est un hydrocarbure fluoré ayant un point d'ébullition de 93°C à 216°C.

24. Article selon la revendication 22 ou 23, dans lequel le système désactivateur de l'antimicrobien est sous forme de particules solides.

25. Article destiné à être utilisé dans les procédés selon les revendications 1 à 21 pour le prélèvement et le traitement d'agents pathogènes microbiens à partir d'un échantillon fluide comprenant:

—un réceptacle contenant un matériau de traitement d'un échantillon qui comprend un système de désactivation d'un facteur antimicrobien selon les revendications 1 à 21.

26. Article selon la revendication 25, dans lequel ledit article comprend un tube flexible allongé avec une première extrémité fermée et une seconde extrémité ouverte; un élément de fermeture amovible pour ladite seconde extrémité; une ampoule écrasable disposée de façon adjacente à ladite extrémité fermée et contenant en son sein un milieu de culture liquide; un corps de matériau absorbant disposé de façon adjacente à ladite ampoule et entre ladite ampoule et ladite extrémité ouverte générale et contenant le système de désactivation du facteur antimicrobien sous forme de particules solides selon les revendications 1 à 21 distribuées en son sein; un tampon comprenant un matériau absorbant contenu à l'une de ses extrémités et disposé de façon adjacente audit corps de matériau absorbant et relié à un organe de prise qui s'étend à partir de ce dernier vers ladite seconde extrémité ouverte.

27. Article selon la revendication 26, dans lequel ledit organe de pris dudit tampon qui s'étend vers la seconde extrémité ouverte est relié audit élément de fermeture amovible.

FIG. 1

FIG. 2

FIG. 3

FIG. 4

CENTRIFUGATION IN ANGLE ROTOR CENTRIFUGE

FIG. 5

FIG. 6

FIG. 7

FIG. 8

CULTURE

FIG. 9

FIG. 10

EP 0 107 070 B1